# EUROPEAN PATENT APPLICATION

(11) **EP 4 218 822 A1**
(43) Date of publication of application: **02.08.2023**
(21) Application number: 21872533.1
(22) Date of filing: 24.09.2021
(51) Int. Cl.: A61K 47/38, A61K 9/06, A61K 9/08, A61K 31/167, A61K 31/196, A61K 31/4422, A61K 38/18, A61K 45/00, A61P 27/16, C07K 14/485

(54) **PHARMACEUTICAL COMPOSITION FOR OTIC ADMINISTRATION**

(30) Priority: 25.09.2020 JP 2020160367
(71) Applicant: Astellas Pharma Inc., Chuo-ku Tokyo 103-8411 (JP)
(72) Inventor: OSADA Mari, Tokyo 103-8411 (JP); KOJIMA Hiroyuki, Tokyo 103-8411 (JP); YOSHIDA Takatsune, Tokyo 103-8411 (JP); YOSHIKAWA Natsuki, Tokyo 103-8411 (JP); NAGAKURA Akira, Tokyo 103-8411 (JP); SATO Luna, Tokyo 103-8411 (JP)
(74) Representative: Hoffmann Eitle
(86) International application number: PCT/JP2021/034972
(87) International publication number: WO 2022/065404

(57) **Abstract**

Provided is a pharmaceutical composition that can be easily administered into the ear and has a function of allowing a drug to be retained and slowly released in the ear, a pharmaceutical composition for otic administration comprising one, or two or more drugs and a water-dispersible fine cellulose composition, in particular, microcrystalline cellulose-carmellose sodium.

SEQUENCE LISTING

<110>  Astellas Pharma Inc.

<120>  Pharmaceutical composition for otic administration

<130>  Application document number

<150>  JP 2020-160367
<151>  2020-09-25

<160>  1

<170>  PatentIn version 3.5

<210>  1
<211>  86
<212>  PRT
<213>  Homo sapiens

<400>  1

Asp Leu Gln Glu Ala Asp Leu Asp Leu Leu Arg Val Thr Leu Ser Ser
1               5                   10                  15

Lys Pro Gln Ala Leu Ala Thr Pro Asn Lys Glu Glu His Gly Lys Arg
            20                  25                  30

Lys Lys Lys Gly Lys Gly Leu Gly Lys Lys Arg Asp Pro Cys Leu Arg
        35                  40                  45

Lys Tyr Lys Asp Phe Cys Ile His Gly Glu Cys Lys Tyr Val Lys Glu
    50                  55                  60

Leu Arg Ala Pro Ser Cys Ile Cys His Pro Gly Tyr His Gly Glu Arg
65                  70                  75                  80

Cys His Gly Leu Ser Leu
                85

## Description

### TECHNICAL FIELD

The present invention relates to a pharmaceutical composition that can be easily administered into the ear and has a function of allowing a drug to be retained and slowly released in the ear.

### BACKGROUND ART

Regarding ear diseases, the number of drug therapy methods is still small, and frequently, the treatment depends on surgical operations. Furthermore, even if a drug effective for the treatment is discovered, when the drug is systemically delivered by oral administration or intravenous administration, there is a risk of a decline in efficacy, adverse side effects, or the like. Therefore, ear drops are mainly used as medicines or pharmaceutical compositions for locally administering a drug into a diseased site of the ear or the vicinity thereof, and retained preparation technology has been developed.

In Patent literature 1 (WO 2011/049958), a sustained release gel preparation utilizing a thermo-reversible gel is reported as the retained preparation technology. It is reported that when a solution obtained by dissolving a polyoxyethylene-polyoxypropylene triblock copolymer, which is a constituent component of the thermo-reversible gel, at a concentration of approximately 14% by weight to approximately 21% by weight is administered onto the round window membrane or the vicinity thereof by means of an injection needle, the solution is warmed in the ear (in Non-Patent literature 1: James M. Chamberlain et al., ANNALS OF ENERGENCY MEDICINE, 1995, 25(1), p. 15-20, it is reported that the temperature inside the external auditory canal is approximately 37°C to approximately 38°C) to gelate, and the gel is retained on the round window membrane or in the vicinity thereof.

In Patent literature 2 (WO 2014/186075), a method of producing a sustained release gel containing a crosslinked copolymer hydrogel of chitosan and polylactide as a constituent component and administering it into the ear is reported. Furthermore, a therapeutic method that uses a syringe to retain on a tympanic membrane perforation is reported. Chitosan and polylactide, which is a hydrolysable crosslinking agent, have characteristics of undergoing chemical crosslinking as a result of mixing immediately before administration or during administration, and changing into the crosslinked copolymer hydrogel within several minutes.

A plurality of pharmaceutical compositions having a function by which a drug is retained and slowly released in the ear has been reported. However, due to strict temperature management, limitation of dosing time, need for special dosing devices, or the like, in order to provide a pharmaceutical composition that can be easily administered into the ear and has a function of allowing a drug to be retained and slowly released in the ear, there is room for further investigation.

### CITATION LIST

### PATENT LITERATURE

[Patent literature 1] WO 2011/049958
[Patent literature 2] WO 2014/186075

### NON-PATENT LITERATURE

[Non-patent literature 1] James M Chamberlain et al., ANNALS OF EMERGENCY MEDICINE, 1995, 25(1), p. 15-20

### SUMMARY OF INVENTION

### TECHNICAL PROBLEM

An object of the present invention is to provide a pharmaceutical composition that can be easily administered into the ear and has a function of allowing a drug to be retained and slowly released in the ear.

### SOLUTION TO PROBLEM

The inventors of the present invention conducted a thorough investigation on a pharmaceutical composition that can be easily administered into the ear and has a function of allowing a drug to be retained and slowly released in the ear, and as a result, the inventors found, in a pharmaceutical composition containing a water-dispersible fine cellulose composition, that the viscosity of the pharmaceutical composition was not affected by temperature under the conditions of 25 to 37°C, which was in the range of room temperature in the medical field to the temperature in the ear; that no special administration device was required; that the maximum dosing pressure at the time of injection was low; that it had retention properties in the ear; that it had sustained release properties; and the like, and completed the present invention.

The present invention relates to the following:
[1] A pharmaceutical composition for otic administration, comprising one, or two or more drugs and a water-dispersible fine cellulose composition.
[2] The pharmaceutical composition of [1], wherein the water-dispersible fine cellulose composition contains microcrystalline cellulose and a water-soluble polymer.
[3] The pharmaceutical composition of [2], wherein the water-soluble polymer is carmellose sodium.
[4] The pharmaceutical composition of [1], wherein the water-dispersible fine cellulose composition is microcrystalline cellulose-carmellose sodium.
[5] The pharmaceutical composition of any one of [1] to [4], further comprising a solvent.
[6] The pharmaceutical composition of [5], wherein the solvent is water.
[7] The pharmaceutical composition of [5] or [6], wherein a ratio of total weight of the water-dispersible fine cellulose composition to the pharmaceutical composition is 1.7% (w/w) to 9.1% (w/w).
[8] The pharmaceutical composition of [5] or [6], wherein a ratio of total weight of the water-dispersible fine cellulose composition to the pharmaceutical composition is 2.9% (w/w) to 9.1% (w/w).
[9] The pharmaceutical composition of any one of [5] to [8], wherein the drug is slowly released, when an insert equipped with a permeable membrane is inserted into a plate well containing 2 mL of phosphate buffered saline at 37°C ± 2°C so that the phosphate buffered saline is divided into an upper layer and a lower layer, 0.5 mL of the pharmaceutical composition according to any one of claims 1 to 8 is added onto the insert, and the drug is dissolved by shaking the plate well at a rate at which a water surface of the phosphate buffered saline shakes.
[10] The pharmaceutical composition of any one of [1] to [9], further comprising a solubilizer.
[11] The pharmaceutical composition of any one of [1] to [10], wherein the drug is one, two or more compounds selected from the group consisting of a small molecule compound, a nucleic acid, and a protein.
[12] The pharmaceutical composition of any one of [1] to [11], wherein the drug is a drug that provides a biological activity of a heparin-binding epidermal growth factor.
[13] The pharmaceutical composition of [12], wherein the drug that provides a biological activity of a heparin-binding epidermal growth factor comprises a protein consisting of the amino acid sequence of SEQ ID NO: 1.
[14] A method of retaining a pharmaceutical composition for otic administration containing one, two or more drugs into the ear by a water-dispersible fine cellulose composition.
[15] The method according to claim 14, wherein the water-dispersible fine cellulose composition is microcrystalline cellulose-carmellose sodium.

### ADVANTAGEOUS EFFECTS OF INVENTION

According to the present invention, there can be provided a pharmaceutical composition that contains one, two or more drugs and a water-dispersible fine cellulose composition, in particular, microcrystalline cellulose-carmellose sodium, and that can be easily administered into the ear and has a function of allowing a drug to be retained and slowly released in the ear.

Furthermore, according to the present invention, there can be provided a method of retaining a pharmaceutical composition for otic administration containing one, two or more drugs into the ear by a water-dispersible fine cellulose composition, in particular, microcrystalline cellulose-carmellose sodium.

### BRIEF DESCRIPTION OF THE DRAWINGS

[Fig. 1] Fig. 1 is a graph showing, in a fluidity test of a microcrystalline cellulose-carmellose sodium (MCC·CMCNa) base liquid and a Pluronic (registered trademark) F-127 (Plu.) base liquid, which was carried out in Test Example 2, the results of measuring the moving distance flowing down the bottom of a stainless-steel square vat tilted at 30 degrees for 60 seconds every 10 seconds.
[Fig. 2] Fig. 2 is a graph showing the results of measuring the static viscosity and dynamic viscosity of MCC·CMCNa base liquids, which was carried out in Test Example 4-2 in order to evaluate the effect of base concentration.
[Fig. 3] Fig. 3 is a graph showing the results of measuring the static viscosity and dynamic viscosity of MCC·CMCNa base liquids, which was carried out in Test Example 5-1 in order to evaluate the effect of DMSO on base liquid viscosity.
[Fig. 4] Fig. 4 is a graph showing the results of measuring the complex viscosity of Plu. base liquids, which was carried out in Test Example 5-2 in order to evaluate the effect of DMSO, which was a solubilizer, on base liquid viscosity.

### DESCRIPTION OF EMBODIMENTS

The present invention relates to a pharmaceutical composition for otic administration comprising one, two or more drugs and a water-dispersible fine cellulose composition, in particular, microcrystalline cellulose-carmellose sodium.

The ear is distinguished into the outer ear, the middle ear, and the inner ear. The outer ear is composed of the pinna, the external auditory canal, and outer membrane of the tympanic membrane. The middle ear is composed of inner membrane of the tympanic membrane, auditory ossicles (malleus, incus, and stapes), tympanic cavity, muscles of auditory ossicles, Eustachian tube, mastoid antrum, and mastoid air cells. The inner ear is composed of the oval window membrane, vestibule, semicircular canal, utricle, saccule, round window membrane, cochlear and the like.

The term "otic administration" as used herein typically means administering a pharmaceutical composition into the external auditory canal, in the vicinity of outer membrane of the tympanic membrane, on the tympanic membrane, in the vicinity of inner membrane of the tympanic membrane, into the tympanic cavity, on the round window membrane, or in the vicinity of the round window, but it is not limited thereto. Preferably it means administering a pharmaceutical composition in the vicinity of inner membrane of the tympanic membrane, into the tympanic cavity, on the round window membrane, or in the vicinity of the round window. The term "pharmaceutical composition for otic administration" as used herein typically means a pharmaceutical composition to be administered into the external auditory canal, in the vicinity of outer membrane of the tympanic membrane, on the tympanic membrane, in the vicinity of inner membrane of the tympanic membrane, into the tympanic cavity, on the round window membrane, or in the vicinity of the round window, but the term is not limited thereto. Preferably it means a pharmaceutical composition to be administered in the vicinity of inner membrane of the tympanic membrane, into the tympanic cavity, on the round window membrane, or in the vicinity of the round window.

Typical forms of the pharmaceutical composition of the present invention include a solid, a powder, a liquid, a paste, or the like, but the form is not limited thereto. The form is preferably a liquid. In connection with this, the term "liquid" as used herein can include a sol, a gel, a suspension, a dispersion, and a lotion, and a preferred liquid is a dispersion. The term "sol" as used herein means a fluid state in which colloidal particles are dispersed in a liquid. The term "gel" as used herein means a state in which the viscosity of the sol increases and the fluidity disappears. When the pharmaceutical composition is in a liquid state and contains a drug, it may be referred to as a "liquid preparation".

The term "water-dispersible fine cellulose composition" as used herein means a composition containing microcrystalline cellulose that easily disperses in water. Examples of the water-dispersible fine cellulose composition used in the present invention include, typically, a composition comprising a mixture in which a water-soluble polymer is attached to or coated on microcrystalline cellulose, but are not limited thereto. The preferred water-dispersible fine cellulose composition is a composition comprising a mixture in which a water-soluble polymer is coated on microcrystalline cellulose. Examples of a method of attaching or coating a water-soluble polymer to microcrystalline cellulose include a method in which microcrystalline cellulose and a water-soluble polymer are mixed, a solvent such as purified water or the like is added to the mixture to prepare a suspension or paste, the suspension or paste is thinly spread and dried by heat treatment or the like, and the dried product is pulverized to obtain powder; a method of drying microcrystalline cellulose that has been fluidized by a heated air flow while spraying a liquid in which a water-soluble polymer is dissolved, and sizing the dried product; and the like, but are not limited thereto.

The term "water-soluble polymer" as used herein means a polymer that has many hydrophilic polar groups along the main chain of the polymer and therefore has the property of being soluble in water as a macromolecule. The water-soluble polymer used in the present invention is not particularly limited, so long as it is pharmaceutical acceptable and improves the dispersibility of microcrystalline cellulose in water. The examples of the water-soluble polymer include, typically, plant-based natural polymers, such as guar gum, carrageenan, karaya gum, locust bin gum, gellan gum, glucomannan, sodium alginate, corn starch, or the like; microbial natural polymers, such as xanthan gum or the like; animal-based natural polymers, such as sodium chondroitin sulfate, sodium hyaluronate, or the like; semi-synthetic polymers, such as carmellose sodium, dextrin, methylcellulose, hydroxyethyl cellulose, hydroxypropyl methylcellulose, cationized guar gum, or the like; and synthetic polymers, such as carboxyvinyl polymer, polyacrylic acid, polyvinylpyrrolidone, polyvinyl alcohol, or the like, but are not limited thereto. Carmellose sodium, karaya gum, dextrin, or xanthan gum is preferable, and carmellose sodium is more preferable.

Carmellose sodium may also be referred to as carboxymethyl cellulose sodium or CMC sodium (hereinafter, may also be referred to as CMCNa), and is a cellulose-based water-soluble polymer. Carmellose sodium can be distinguished by the viscosity (25°C, 60 rpm) and the degree of etherification (degree of substitution) in a 1% aqueous solution. The viscosity is typically 20 to 500 Pa s, and preferably 50 to 300 Pa·s. The degree of substitution is typically 0.5 to 2.0, and preferably 0.55 to 1.1. Examples of a preferable commercially available product of carmellose sodium include CMC Daicel 1120, 1130, 1140, 1150, 1160 (manufactured by Daicel), Sunrose (registered trademark) F-10MC, F-30MC (manufactured by Nippon Paper Industries), and the like, but are not limited thereto. CMC Daicel 1150 (manufactured by Daicel) is preferable.

The water-dispersible fine cellulose composition is preferably microcrystalline cellulose-carmellose sodium (hereinafter also referred to as MCC·CMCNa). Microcrystalline cellulose-carmellose sodium may also be referred to as "microcrystalline cellulose and carmellose sodium", but is a mixture of crystalline cellulose and sodium carmellose for easy microdispersion. More particularly, it is typically a product in which sodium carmellose is attached to or coated on microcrystalline cellulose, and preferably a product in which sodium carmellose is coated on microcrystalline cellulose. The content of microcrystalline cellulose in microcrystalline cellulose-carmellose sodium is typically 50%(w/w) to 95%(w/w), preferably 70%(w/w) to 94%(w/w), and more preferably 80%(w/w) to 93%(w/w). Each upper limit and each lower limit can be arbitrarily combined, for example, 50%(w/w) to 93%(w/w) or the like, if desired. The content of carmellose sodium in microcrystalline cellulose-carmellose sodium is typically 5%(w/w) to 50%(w/w), preferably 6%(w/w) to 30%(w/w), and more preferably 7%(w/w) to 20%(w/w). Each upper limit and each lower limit can be arbitrarily combined, for example, 5%(w/w) to 20%(w/w) or the like, if desired.

Examples of a commercially available product of microcrystalline cellulose-carmellose sodium include CEOLUS (registered trademark) RC-591NF (manufactured by Asahi Kasei), AVICEL (registered trademark) RC-591, RC-581, BV1518 (manufactured by FMC Biopolymer), but are not limited thereto.

When the pharmaceutical composition is in a solid or powder state, the amount of the water-dispersible fine cellulose composition, in particular, microcrystalline cellulose-carmellose sodium, is, for example, 0.03 mg to 60 mg per dose unit, preferably 0.3 mg to 45 mg, more preferably 0.6 mg to 37.5 mg, still more preferably 0.9 mg to 30 mg, still more preferably 1.5 mg to 22.5 mg, still more preferably 2.2 5mg to 18 mg, still more preferably 3 mg to 15 mg, and still more preferably 3 mg to 9 mg. Each upper limit and each lower limit can be arbitrarily combined, for example, 0.03 mg to 45 mg or the like, if desired.

Typical examples of a "solvent" used in the pharmaceutical composition of the present invention, or a solvent for dissolving or dispersing the pharmaceutical composition of the present invention include water (purified water, water for injection, other pharmaceutically acceptable water), a mixture of water and a water-miscible solvent such as alkanol having 1 to 7 carbon atoms, a dextrose aqueous solution, and the like, but the solvent is not limited thereto. The solvent is preferably water.

When the pharmaceutical composition is in a form of a solution, a dispersion, a suspension, a paste, or a lotion, the concentration of the water-dispersible fine cellulose composition, in particular, microcrystalline cellulose-carmellose sodium, used in the present invention is, with respect to the pharmaceutical composition, for example, typically 1.7%(w/w) to 9.1%(w/w), preferably 1.9%(w/w) to 9.1%(w/w), more preferably 2.4%(w/w) to 9.1%(w/w), still more preferably 2.9%(w/w) to 9.1%(w/w) , still more preferably 2.9%(w/w) to 8.3%(w/w) , still more preferably 2.9%(w/w) to 6.5%(w/w) , still more preferably 2.9%(w/w) to 5.7%(w/w), and still more preferably 2.9%(w/w) to 4.8%(w/w). Each upper limit and each lower limit can be arbitrarily combined, for example, 2.9%(w/w) to 8.3%(w/w) or the like, if desired.

The term "base" as used herein means, among the constituent components of the pharmaceutical composition, a pharmaceutical additive having mainly a function of allowing a drug to be retained and slowly released in the ear, or a combination of pharmaceutical additives having mainly a function of allowing a drug to be retained and slowly released in the ear. More particularly, the base means, for example, a water-dispersible fine cellulose composition, a water-dispersible fine cellulose composition and water, or the remaining pharmaceutical additives after removing the drug from the pharmaceutical composition, but is not limited thereto. In the case where the base is in a liquid state, it is sometimes called "base liquid".

The term "gelation" or "gel formation" as used herein means that a pharmaceutical composition or a base having fluidity comes to lose fluidity temporarily or continuously, and the term "gel-forming ability" means a property of gelating. If the pharmaceutical composition flows too much, the pharmaceutical composition will be cleared from the ear canal and eustachian tube, it is preferable that the pharmaceutical composition or the base rapidly forms a gel temporarily or continuously after otic administration. A method of evaluating the presence or absence of gel formation and the gel-forming ability may be a method of dropping 300 µL, 200 µL, 100 µL, or 50 µL of a pharmaceutical composition or a base onto a plastic Petri dish or into a test tube, covering the Petri dish or the test tube with a lid, subsequently placing the Petri dish or the test tube on the water surface of a water bath or on a heat block set to 37°C, taking out the plastic Petri dish or the test tube after 10 seconds, after 15 seconds, after 20 seconds, after 30 seconds, after 1 minute, after 5 minutes, after 10 minutes, after 15 minutes, or after 30 minutes from the beginning of placement, immediately inverting the Petri dish or the test tube, and checking the behavior of the pharmaceutical composition or the base by visual inspection for 10 seconds from the beginning of inversion (hereinafter also referred to as a gel-forming ability test), but the method is not limited thereto. In a sample that has been placed for 10 seconds, for 15 seconds, for 20 seconds, for 30 seconds, for 1 minute, for 5 minutes, or for 10 minutes on the water surface of a water bath set to 37°C in the gel-forming ability test, typically "dropping" and "flowing horizontally" are not observed for at least 5 seconds from the inversion, and preferably "dropping" and "flowing horizontally" are not observed for at least 10 seconds from the inversion.

In this connection, when the pharmaceutical composition or the base is a solid, powder, or the like, and the pharmaceutical composition or the base is dissolved or dispersed in an appropriate amount or a specified amount of purified water or other pharmaceutically acceptable solvent before administration into the ear and is administered into the ear, the fluidity should be evaluated after dissolving or dispersing it in an appropriate amount or a specified amount of purified water or other pharmaceutically acceptable solvent (hereinafter, when the pharmaceutical composition or the base cannot be appropriately evaluated due to a solid or powder state in the evaluation method of the pharmaceutical composition or the base described in the present specification, each item should be evaluated after dissolving or dispersing it in an appropriate amount or a specified amount of purified water or other pharmaceutically acceptable solvent).

It is preferable that, after administration into the ear, the pharmaceutical composition of the present invention does not flow immediately or has low fluidity. Examples of a method of evaluating the fluidity of the pharmaceutical composition of the present invention or the base include a method in which a stainless-steel square vat (for example, 18-8 shallow square vat, manufactured by Clover) is placed in a chromatographic chamber (M-600FN, manufactured by TAITEC) with a set temperature of 37°C so that the angle between the bottom surface of the square vat and the horizontal is 30 degrees, and 0.5 mL of the pharmaceutical composition or the base is gently dropped onto the bottom surface of the square vat, and the moving distance of the pharmaceutical composition or the base flowing down the bottom surface of the square vat for 60 seconds after the dropping is measure (hereinafter, may also be referred to as a fluidity test), but are not limited thereto. When the fluidity test is performed, the shorter the moving distance for 60 seconds after dropping, the more the pharmaceutical composition can avoid clearance from the ear canal or the eustachian tube, and the retention in the ear can be expected. The preferred moving distance for 60 seconds after dropping is typically 3.0 cm or less, preferably 2.5 cm or less, more preferably 2.0 cm or less, still more preferably 1.8 cm or less, and still more preferably 1.5 cm or less. The lower limit is 0 cm.

The term "viscosity" or "static viscosity" as used herein means a viscosity obtained using a viscoelasticity measuring device (hereinafter referred to as rheometer) by dynamic measurement, and it is characterized in that the shear rate at the time of measurement is set to a speed at which the sample can be regarded as a stationary state. It can be obtained by, for example, a method in which the dynamic measurement is carried out using a rheometer manufactured by Anton Paar (MCR302, a plate: MEASURING CONE CP50-1, a hood: H-PTD200, a gap: 0.3 mm), under the measuring conditions of a sample volume of 1 mL, a shear rate of 0.01 (1/sec.), a normal force of 0 N, a torque reliability range of 1 µN·m or more, and a temperature of 37°C (hereinafter, may also be referred to as a static viscosity measurement test), but it is not limited thereto. If the torque reliability range is not exceeded in the static viscosity measurement test, it is regarded as LOQ (Limit of Quantitation).

In the pharmaceutical composition of the present invention, it is preferable that the static viscosity is rapidly increased so as not to be cleared from the ear canal or the eustachian tube after administration into the ear using a syringe and an injection needle usually used in the medical field. A method of measuring static viscosity is not particularly limited, but for example, typically, the viscosity after 10 seconds, after 20 seconds, or after 30 seconds from the beginning of the static viscosity measurement test, or the viscosity after 10 seconds, after 20 seconds, after 30 seconds, after 60 seconds, after 90 seconds, after 120 seconds, after 150 seconds, after 180 seconds, after 210 seconds, after 240 seconds, after 270 seconds, or after 300 seconds from the change of the measurement conditions of the dynamic viscosity measurement test described below to the measurement conditions of the static viscosity measurement test is typically 10 Pa s or more, preferably 20 Pa s or more, more preferably 30 Pa s or more, still more preferably 50 Pa s or more, and still more preferably 100 Pa·s or more. Preferably, the viscosity after 120 seconds from the change of the measurement conditions of the dynamic viscosity measurement test described below to the measurement conditions of the static viscosity measurement test is typically 10 Pa·s or more, preferably 20 Pa s or more, more preferably 30 Pa s or more, still more preferably 50 Pa s or more, and still more preferably 100 Pa·s or more. More preferably, the viscosity after 60 seconds from the change of the measurement conditions of the dynamic viscosity measurement test described below to the measurement conditions of the static viscosity measurement test is typically 10 Pa·s or more, preferably 20 Pa s or more, more preferably 30 Pa s or more, still more preferably 50 Pa s or more, and still more preferably 100 Pa s or more. In this connection, since if the viscosity is too high, there is a risk that the retained material may come off from the wall surface in the ear after administration and be cleared from the ear canal or the eustachian tube, the upper limit of the viscosity is typically 20000 Pa·s or less.

The term "dynamic viscosity" as used herein means a viscosity obtained using a rheometer by dynamic measurement, and it is characterized in that the shear rate at the time of measurement is set to a speed at which the sample can be regarded as a fluidized state. It can be obtained by, for example, a method in which the dynamic measurement is carried out using a rheometer manufactured by Anton Paar (MCR302, a plate: MEASURING CONE CP50-1, a hood: H-PTD200, a gap: 0.3 mm), under the measuring conditions of a sample volume of 1 mL, a shear rate of 1000 (1/sec.), a normal force of 0 N, a torque reliability range of 1 µN·m or more, and a temperature of 37°C (hereinafter, may also be referred to as a dynamic viscosity measurement test), but it is not limited thereto. If the torque reliability range is not exceeded in the dynamic viscosity measurement test, it is regarded as LOQ.

In the pharmaceutical composition of the present invention, it is preferable that administration into the ear can be carried out using a syringe and an injection needle usually used in the medical field. The dynamic viscosity of the pharmaceutical composition affects the maximum dose pressure during injection administration, and there is a risk that a high dynamic viscosity may make it difficult to administer it into the ear easily, and therefore, it is preferable that the dynamic viscosity is low. That is, in the pharmaceutical composition of the present invention, it is preferable that the viscosity of the pharmaceutical composition decreases when a shearing force is applied. A method of measuring dynamic viscosity is not particularly limited, but when the dynamic viscosity is measured by the dynamic viscosity measurement test, the viscosity of the pharmaceutical composition, the base, or the base liquid is, typically during the dynamic viscosity measurement test, typically less than 10 Pa·s, preferably less than 5 Pa·s, more preferably less than 1 Pa·s, still more preferably less than 0.25 Pa s, and still more preferably 0.20 Pa s. Preferably, at the time after 30 seconds from the change to the measurement conditions of the dynamic viscosity measurement test, it is typically less than 10 Pa s, preferably less than 5 Pa s, more preferably less than 1 Pa s, still more preferably less than 0.25 Pa·s, and still more preferably 0.20 Pa·s. The lower limit is 0 Pa s.

The term "complex viscosity" as used herein means a viscosity obtained by dynamic measurement using a rheometer. When a polymer constituting a thermoreversible gel is used as a base, the viscosity of the base or the pharmaceutical composition can be evaluated. As a method of measuring complex viscosity, a method using a rheometer can be exemplified, but it is not limited thereto. A method of measuring the complex viscosity of the pharmaceutical composition or the base using a rheometer may be, for example, a method in which the measurement is carried out using a rheometer (MCR302, manufactured by Anton Paar) equipped with a plate (MEASURING CONE CP25-2), a hood (H-PTD200), and a gap of 0.3 mm, under the conditions of a sample volume of 0.3 mL, a shear strain of 5%, an angular frequency of 50 rad/sec., a normal force of 0 N, and a torque reliability range of 1 µN·m or more (hereinafter also referred to as a complex viscosity measurement test), but is not limited thereto. If the torque reliability range is not exceeded under the complex viscosity measurement test conditions, it is regarded as LOQ.

In the pharmaceutical composition of the present invention, it is preferable that the maximum dosing pressure (maximum pressurization, maximum pressing pressure) when extruding the pharmaceutical composition is low so that the pharmaceutical composition can be easily administered into the ear using a syringe and an injection needle. Examples of a method of measuring maximum dosing pressure include a method using a load cell, but are not limited thereto. The method of measuring maximum dosing pressure using a load cell may be, for example, a method in which a load cell (LC2-3305B-200N, contact area: 25 mm², manufactured by YAMADEN) is set in a creepmeter (RHEONER2 CREEPMETER RE2-3305C, manufactured by YAMADEN) to prepare a measuring device; an appropriate amount of the pharmaceutical composition or base is filled in a luer lock syringe (SOFT-JECT S5010-LL, manufactured by Henke sass wolf), and a 25G injection needle (25G × 60 mm, manufactured by TOP) is attached; air bubbles are removed, and the filling liquid volume is adjusted to 0.5 mL to prepare a sample for measurement; the sample is set in the measuring device; and the force required to push it at a speed of 1 mm/sec. in the vertical direction is measured (hereinafter, may also be referred to as dosing pressure measurement test), but it is not limited thereto. The measurement temperature is not particularly limited, so long as it is room temperature or within the range of approximately 37°C to 38°C. The preferred maximum dosing pressure in the dosing pressure measurement test is typically 10 N or less, preferably 9 N or less, more preferably 7.2 N or less, still more preferably 6 N or less, and still more preferably 5 N or less. The lower limit of the maximum dosing pressure is 0 N.

The "sol-gel transition point" as used herein means the temperature at the time when a pharmaceutical composition is converted from a sol state to a gel state. A method for evaluating the sol-gel transition point may be, for example, a method using a rheometer or the like, but is not limited thereto. In the present specification, when the complex viscosity of the pharmaceutical composition or the base is measured using, for example, a rheometer (MCR302) manufactured by Anton Paar, the storage modulus (G'), which is a solid element, and the loss modulus (G"), which is a liquid element, are simultaneously digitized, and when G' < G" is designated as a sol state, while G' > G" is designated as a gel state, the temperature at the time point when G' = G" is achieved can be designated as the sol-gel transition point.

The wording "a drug is slowly released" or "sustained release properties" means that, after administration of a pharmaceutical composition, a drug continues to dissolve for a certain period of time, or the properties that continue to dissolve. The dissolution rate is not particularly limited, but it is preferable that the drug is slowly released, because the purpose of sustained drug release of the drug is to reduce the number of administrations, and/or, to maintain the drug effect and to reduce side effects by maintaining a constant drug concentration at a target site.

Examples of a method of evaluating sustained release properties of a drug include, typically as a method referring to the method described in "Dose-dependent sustained release of dexamethasone in inner ear cochlear fluids using a novel local delivery Approach (Xiaobo Wang, 2009 Audiol Neurotol 14 : 393-401", a method in which 2 mL of dissolution test liquid is added to a plate well (for example, Transwell (registered trademark) or Snapwell (trademark) (6 well, 12 mm diameter inserts, 0.4 µm pore size, manufactured by Corning)) capable of dividing into the upper layer and the lower layer by an insert with a permeable membrane (insert membrane), and is heated to 37°C ± 2°C together with the plate well; the insert membrane is inserted into the plate well, and 0.5 mL of a pharmaceutical composition containing one, or two or more drugs is added onto the insert; the drug is dissolved by heating at 37°C ± 2°C and shaking the plate well at a rate at which a water surface of the dissolution test liquid shakes (for example, 100 rpm in a chromatographic chamber M-600FN, manufactured by TAITEC); each drug concentration in the lower layer of the insert membrane is quantified, for example, with respect to at least 3 time points of 1, 6, and 24 hours after the start of the dissolution test, 1, 4, and 8 hours after the start of the dissolution test, or 1, 24, and 48 hours after the start of the dissolution test; and each dissolution rate is calculated from the drug concentration (hereinafter, may be referred to as dissolution test), or a method in which 2 mL of a pharmaceutical composition is added to a plate well (for example, CELLSTAR (trademark), 6-well, plate, SC (manufactured by Greiner)) so that the pharmaceutical composition spreads evenly on the bottom of the well; the dissolution of a drug from the pharmaceutical composition is initiated by gently adding 3 mL of a dissolution liquid thereon along the wall surface of the well; the drug concentration in the dissolution liquid is quantified, for example, with respect to at least 3 time points of 1, 6, and 24 hours after the start of the dissolution, 1, 4, and 8 hours after the start of the dissolution, or 1, 24, and 48 hours after the start of the dissolution; and the dissolution rate is calculated from the drug concentration, but are not limited thereto. The method by the dissolution test is preferred.

As the method for quantifying the drug, the most suitable analysis method for quantifying the drug may be used, and examples of the method include high performance liquid chromatography (HPLC), fluorometer, or the like, but are not limited thereto. The dissolution test liquid is not particular limited, so long as the dissolution properties can be suitably evaluated. If the solubility of the drug is pH independent, examples of the dissolution test liquid include phosphate buffered saline (hereinafter, may also be referred to as PBS: phosphate buffered salts) such as D-PBS(-) or the like, but are not limited thereto. If the solubility of the drug depends on pH or if the solubility of the drug is low, a preferable dissolution test liquid (for example, a solvent capable of dissolving 100% of a certain amount of drug) may be used for each drug. For example, a surfactant (for example, polysorbate 20 or the like) can be added to physiological saline or the like or a solvent at a low concentration, but it is not limited thereto.

The wording "a drug is slowly released" as used herein means that, although the dissolution rate of a drug with respect to a suitable dissolution test time that depends on ear diseases and/or the drug varies, for example, the dissolution rate of a drug at the time point of 1 hour after the start of the dissolution test is typically 40% or less, preferably 30% or less, more preferably 20% or less, still more preferably 15% or less, and still more preferably 12% or less. Furthermore, it means that, for example, the dissolution rate of a drug at the time point of 6 hours after the start of the dissolution test is typically 70% or less, preferably 60% or less, more preferably 50% or less, still more preferably 40% or less, and still more preferably 35% or less. Furthermore, it means that, for example, the dissolution rate of a drug at the time point of 24 hours after the start of the dissolution test is typically 85% or less, preferably 75% or less, more preferably 70% or less, and still more preferably 60% or less.

The term "retention" or "retention properties" as used herein means that the drug contained in a pharmaceutical composition administered into the ear avoids the clearance of the external auditory canal and the eustachian tube, and remains at the administration site temporarily or continuously, or the properties that remain at the administration site temporarily or continuously. A method of evaluating the retention properties is not particularly limited. Examples of the method include PET (positron emission tomography) imaging, SPECT (single photon emission computed tomography) imaging, a test using a fluorescent dye, MRI (magnetic resonance imaging), or the like, but are not limited thereto. The test using a fluorescent dye as used herein means a test in which, for example, the pharmaceutical composition containing a drug in which a fluorescent dye is encapsulated or labeled is administered to an administration site in the ear, and the residual amount of fluorescent dye in the ear after a certain period of time is quantitatively determined. The residual rate can be calculated from the administered amount and the residual amount.

With respect to the residual rate of the pharmaceutical composition of the present invention, an appropriate residual rate varies according to ear diseases and/or drugs. When the retention properties of the pharmaceutical composition of the present invention are evaluated by the above method, for example, the period until the residual rate after administration becomes 50% or less is, typically 6 hours or more, preferably 12 hours or more, more preferably 1 day or more, still more preferably 2 days or more, still more preferably 3 days or more, still more preferably 5 days or more, still more preferably 6 days or more, still more preferably 7 days or more, still more preferably 10 days or more, still more preferably 12 days or more, and still more preferably 14 days or more. The period until the residual rate after administration becomes 20% or less is, typically 6 hours or more, preferably 12 hours or more, more preferably 1 day or more, even more preferably 2 days or more, still more preferably 3 days or more, still more preferably 5 days or more, still more preferably 6 days or more, still more preferably 7 days or more, still more preferably 10 days or more, still more preferably 12 days or more, still more preferably 14 days or more, still more preferably 21 days or more, and still more preferably 28 days or more. The case that shows at least the retention properties as described above is included in the case "having retention properties" as used herein, but it is not limited thereto.

With respect to the number of administrations of the pharmaceutical composition of the present invention, an appropriate number varies according to ear diseases and/or drugs. The number (frequency) is typically twice per day, preferably once per day, more preferably once every 2 days, still more preferably once every 3 days, still more preferably once every 4 days, still more preferably once every 5 days, still more preferably once every 6 days, still more preferably once every 7 days, still more preferably once every 10 days, still more preferably once every 12 days, still more preferably once every 14 days, still more preferably once every 21 days, and still more preferably once every 28 days, but it is not limited thereto.

The dosage of the pharmaceutical composition is not particularly limited, as long as it can be administered to a patient. The dosage is typically an amount that can be administered into the ear, preferably an amount necessary to provide a useful contact with the tympanic membrane, or preferably an amount that can be administered into the tympanic cavity. The dosage is typically 1 µL to 2,000 µL, preferably 10 µLto 1,500 µL, more preferably 20 µL to 1,250 µL, still more preferably 30 µL to 1,000 µL, still more preferably 40 µL to 750 µL, still more preferably 50 µL to 600 µL, still more preferably 50 µL to 500 µL, and still more preferably 50 µL to 300 µL. Each upper limit and each lower limit can be arbitrarily combined, for example, 1 µL to 1,500 µL or the like, if desired.

The pharmaceutical composition of the present invention comprises one, two or more drugs. The "drug" used in the present invention is typically an active ingredient needed to treat ear diseases, and preferably a small molecule compound, a nucleic acid, or a protein.

The small molecule compound is a compound having a molecular weight of less than 500. Typical examples of the small molecule compound include acetaminophen, diclofenac sodium, nicardipine hydrochloride, or the like, but are not limited thereto. The nucleic acid is a polymer compound in which nucleotides consisting of bases, sugars, and phosphates are linked by phosphodiester bonds. The protein is a polymer compound formed by chain polymerizing L-amino acids. Typical examples of the protein include an enzyme, a ligand for a target receptor, a receptor itself, or the like, but are not limited thereto. The protein is preferably a ligand for a target receptor, such as a drug that provides the biological activity of a heparin-binding epidermal growth factor.

Examples of the drug that provides the biological activity of a heparin-binding epidermal growth factor used in the present invention include HB-EGF (Heparin-Binding Epidermal Growth Factor-like growth factor), human HB-EGF, recombinant human HB-EGF, or the like, but are not limited thereto. It is typically HB-EGF, preferably human HB-EGF, and more preferably recombinant human HB-EGF.

The drug that provides the biological activity of a heparin-binding epidermal growth factor used in the present invention is typically selected from the following proteins:
a) a protein comprising an amino acid sequence in which 1 to 10 amino acids, 1 to 5 amino acids, 1 to 3 amino acids, or 1 amino acid are deleted, substituted, inserted, and/or added in the amino acid sequence of SEQ ID NO: 1, and having a proliferation and/or a migration on keratinocytes and fibroblasts;
b) a protein comprising an amino sequence having a 60% or more, 75% or more, 85% or more, 90% or more, 95% or more, or 100% identity with the amino acid sequence of SEQ ID NO: 1, and having a proliferation and/or a migration on keratinocytes and fibroblasts; and
c) a protein consisting of the amino acid sequence of SEQ ID NO: 1.

The protein consisting of the amino acid sequence of SEQ ID NO: 1 is preferable.

The drug that provides the biological activity of a heparin-binding epidermal growth factor used in the present invention can be prepared by a person skilled in the art, based on the sequence information disclosed in the present specification, and using methods known in the art (for example, a method described in WO 2014/186075).

When the drug that provides the biological activity of a heparin-binding epidermal growth factor is used, in order to suppress aggregation of the drug that provides the biological activity of a heparin-binding epidermal growth factor, structural change of the drug that provides the biological activity of a heparin-binding epidermal growth factor, or the like, pharmaceutical additives may be appropriately added alone or in combination of two or more in appropriate amounts within the range where the drug effect is exerted.

The pharmaceutical composition of the present invention can be used for the treatment of ear diseases, for example, chronic tympanic membrane perforation or the like, but it is not limited thereto.

In the pharmaceutical composition of the present invention, pharmaceutical additives may be appropriately added alone or in combination of two or more in appropriate amounts, if desired, within the range where the desired effects of the present invention are exerted. Examples of the pharmaceutical additives include a buffer such as PBS or the like; a pH adjuster such as diluted hydrochloric acid, sodium hydroxide, or the like; an isotonic agent; a solubilizer; an antioxidant; a preservative; a surfactant such as polysorbate 20 or the like; or the like, but are not limited thereto.

Examples of the solubilizer include dimethyl sulfoxide (hereinafter sometimes referred to as DMSO), ethanol, dichloromethane, acetone, propylene glycol, polyethylene glycol, glycerol, benzyl alcohol, N,N-dimethylacetamide, or the like, but are not limited thereto. DMSO is preferable.

When the solubilizer is added to the pharmaceutical composition of the present invention, the concentration of the solubilizer used in the present invention is, with respect to the pharmaceutical composition, for example, typically 0.01%(v/v) to 50%(v/v), preferably 0.1%(v/v) to 30%(v/v), more preferably 1%(v/v) to 10%(v/v), and still more preferably 1%(v/v) to 5%(v/v). Each upper limit and each lower limit can be arbitrarily combined, for example, 0.01%(v/v) to 30%(v/v) or the like, if desired.

The method of producing the pharmaceutical composition of the present invention will be described below, but includes known methods comprising steps such as drug preparation, base dispersion, pH adjustment, filling, sterilization, freeze-drying of drug and base, final mixing of drug and base, final sterilization, or the like.

### <Drug preparation step>

In the drug preparation step, the apparatus and means are not particularly limited, as long as the drug can be dissolved, suspended, or dispersed in an ordinary pharmaceutical manner. Examples of the method include a method of dissolving, suspending, or dispersing the drug in purified water or a buffer such as PBS or the like as a solvent, but it is not limited thereto. When the drug is a poorlysoluble drug, a solubilizer such as DMSO or the like may be added for drug solubilization, but it is not limited thereto. When the drug is a protein or the like, a surfactant such as polysorbate 20 or the like may be added in order to prevent aggregation and/or to prevent adsorption to the container, but it is not limited thereto.

### <Base dispersion and pH adjustment steps>

In the base dispersion step, the apparatus and means are not particularly limited, as long as the water-dispersible fine cellulose composition, in particular, microcrystalline cellulose-carmellose sodium can be dispersed in an ordinary pharmaceutical manner. Examples of the method include a method of obtaining a base dispersion by adding the water-dispersible fine cellulose composition, in particular, microcrystalline cellulose-carmellose sodium to a solvent such as purified water or the like, and dispersing it by agitating with a stirrer, but it is not limited thereto. The base dispersion may be pulverized using a wet-type super atomizer in order to reduce viscosity during administration and improve viscosity after administration, but it is not limited thereto. If desired, a pH adjustor such as diluted hydrochloric acid, sodium hydroxide, or the like can be added to adjust the pH within the range where the desired effects of the present invention are achieved, but it is not limited thereto.

### <Filling and sterilization steps>

In the filling step and the sterilization step, the apparatus and means are not particularly limited, as long as the method used is a usually pharmaceutically acceptable filling and sterilization method. Examples of the method include a method in which the dispersed base liquid is dry heat sterilized, and is filled in a container such as a vial or the like, or a device or the like, but it is not limited thereto. The term "sterilization" used herein means killing or removing microorganisms existing in a pharmaceutical composition, a container, a device or the like. Examples of the sterilization method include dry heat sterilization, heat sterilization, high pressure steam sterilization, chemical sterilization, radiation sterilization, or filter sterilization, but it is not limited thereto.

### <Drug freeze-drying step>

The dissolved drug solution and the base dispersion can be frozen or freeze-dried. In the drug freeze-drying step, the apparatus and means are not particularly limited, as long as the method used is a usually pharmaceutically acceptable freeze-drying method.

### <Step of final mixing of drug and base>

The pharmaceutical composition of the present invention can be provided as a frozen dispersion, a refrigerated dispersion, or freeze-dried product in a state where the drug and the base are mixed, or as a frozen solution, a refrigerated solution, or freeze-dried product of the drug, and the base dispersion, which are filled in separate containers, but it is not limited thereto. In the case where the drug and the base solution are provided in separate containers, the pharmaceutical composition for otic administration of the present invention may be prepared, for example, by a method of adding the base dispersion to a vial containing the drug and mixing them, or a method of adding each from each vial containing the drug or the base dispersion to another empty vial and mixing them, but it is not limited thereto.

### <Final sterilization step>

The pharmaceutical composition for otic administration of the present invention can comprise, in its manufacturing process, the final sterilization step, for the purpose of providing a preparation that does not contain microorganisms causing infectious diseases, or a safe preparation. The term "sterilization" used herein means killing or removing microorganisms existing in a pharmaceutical composition for otic administration, a container, a device or the like. The sterilization method is not particularly limited, so long as the method is appropriately changed according to the stability of the drug. As an embodiment, examples of the method include dry heat sterilization, heat sterilization, high pressure steam sterilization, chemical sterilization, radiation sterilization, or filter sterilization, and as an embodiment, the method is filter sterilization.

The container for filling the pharmaceutical composition of the present invention can be selected according to the purpose of use. The container as used herein is not particularly limited, as long as it can be sealed. Examples of the container include a vial, an ampule, a syringe, a bottle-like container with large capacity, or the like, but it is not limited thereto.

The pharmaceutical composition of the present invention can be delivered to a treatment site using a device. The term "device" as used herein means medical equipment or an apparatus that delivers a pharmaceutical composition to a treatment site, and can be selected according to the treatment site. Examples of the device typically include a device in which a syringe (including a disposable syringe) or the like in a form of a prescribed dose is filled with a pharmaceutical composition, and equipped with a needle, a cannula, or a catheter; a double barrel syringe for delivering two liquids simultaneously or while mixing;a spray capable of delivering a pharmaceutical composition to the treatment site by spraying; a pump-type device in which the tip of a catheter connected to an osmotic pump is placed near the round window membrane, and the drug liquid is continuously administered onto the round window membrane from the osmotic pump embedded in the posterior part of the pinna; or the like, but it is not limited thereto. The preferred device is a syringe equipped with a needle. The length and thickness of the needle, cannula, or catheter can be appropriately selected according to the treatment site and a patient. In the case of an administration method in which the tympanic membrane is penetrated when locally administering the drug to or near the diseased site of the ear, it is preferred that a needle, cannula, or catheter of typically 25 to 30 gauge is selected, because a hole opens in the tympanic membrane after administration.

The device can also be used as a container. For example, the pharmaceutical composition of the present invention can be provided as a prefilled syringe liquid preparation or an auto-injector liquid preparation in which a syringe is prefilled with a solution or the like. These eliminate the need for operations such as a dissolution operation and may enable more prompt response in the medical field.

As the material of the container, device, or the like, examples include glass, plastic, or the like, but it is not limited thereto. The container or device can be surface-treated, and can be treated with silica coating, silicone coating, sulfur treatment, various low-alkaline treatments, or the like, but it is not limited thereto.

The present invention includes a method of retaining a pharmaceutical composition for otic administration containing one, two or more drugs into the ear by a water-dispersible fine cellulose composition. The present invention includes a method of retaining a pharmaceutical composition for otic administration containing one, two or more drugs into the ear by microcrystalline cellulose-carmellose sodium.

With respect to the terms "otic administration", "for otic administration", "water-dispersible fine cellulose composition", "microcrystalline cellulose-carmellose sodium", "drug", and "retention", which are used in the method of the present invention, the explanations therefor described in the pharmaceutical composition of the present invention can be directly applied.

With respect to the content of each component and the blending method in the method, which is the present invention, of retaining a pharmaceutical composition for otic administration containing one, two or more drugs into the tympanic cavity by a water-dispersible fine cellulose composition, the explanations described in the pharmaceutical composition of the present invention can be directly applied.

### EXAMPLES

The present invention will now be further illustrated by, but is by no means limited to, the following Examples.

In the Examples below, Ceolus (registered trademark) RC A591NF (MCC content: 80% or more, CMCNa content: 8.3-13.7%)(manufactured by Asahi Kasei), which was microcrystalline cellulose-carmellose sodium; Pluronic (registered trademark) F-127 (hereinafter also referred to as Plu.)(manufactured by BASF), which was a poly(oxyethylene)/poly(oxypropylene) triblock copolymer; sodium hyaluronate (manufactured by ACROS ORGANICS); KIMICA Algin I-5 (manufactured by KIMICA), which was sodium alginate; CMC Daicel 1150 (manufactured by Daicel), which was CMC-Na; Metolose SM-4000 (manufactured by Shin-Etsu Chemical), which was methylcellulose; HEC Daicel SE900 (manufactured by Daicel), which was hydroxyethyl cellulose (HEC); HPC-H (manufactured by Nippon Soda), which was hydroxyethyl cellulose (HPC); Metolose 60SH-4000 (manufactured by Shin-Etsu Chemical), which was hydroxypropyl methylcellulose (HPMC); Kollidon 12PF (manufactured by BASF), which was polyvinylpyrrolidone; GENU HM pectin USP-H (manufactured by Sansho), which was pectin; Carbopol 971P NF (manufactured by Lubrizol); GENUVISCO PJ-JPE (manufactured by Sansho), which was carrageenan; Karaya gum matsu M-150 (manufactured by DSP GOKYO FOOD & CHEMICAL), which was karaya (Sterculia urens) gum; Xanthan gum PA (manufactured by Ina Food Industry), which was xanthan gum; DMSO (manufactured by Kanto Chemical); polysorbate 20 (manufactured by Kanto Chemical) Fluorescein isothiocyanate-dextran, average mol wt 10,000 (manufactured by Sigma-Aldrich) as FITC dextran (10 kDa); Fluorescein isothiocyanate-dextran, average mol wt 70,000 (manufactured by Sigma-Aldrich) as FITC dextran (70 kDa); Fluorescein isothiocyanate-dextran, average mol wt 150,000 (manufactured by Sigma-Aldrich) as FITC dextran (150 kDa)(hereinafter dextran is also referred to as DEX); acetaminophen (manufactured by Yamamoto Corporation); diclofenac sodium (manufactured by FUJIFII,M); nicardipine hydrochloride (manufactured by Tokyo Chemical Industry); and physiological saline (Otsuka Pharmaceutical); were used. Two types of PBS were used in the Examples below. These are 1×PBS (pH 7.4) (manufactured by Gibco) and D-PBS(-) (manufactured by FUJIFII,M). In the Examples, when the concentrations of MCC·CMCNa and Plu. are shown only with the unit "%", it means "%(w/w)".

### Experiment 1: Evaluation of gel forming ability

### <Preparation Example 1-1> Preparation of 3.8% MCC CMCNa base liquid

After 400 mg of MCC·CMCNa was weighed, it was added to 10 g of purified water and dispersed to prepare a 3.8% MCC·CMCNa base liquid (Example 1).

### <Preparation Example 1-2> Preparation of 17.0% Plu.-PBS base liquid

After 3.4 g of Plu. was weighed, it was added to 16.6 g of 1×PBS, and dissolved at approximately 4°C to prepare a 17.0% Plu.-PBS base liquid (Example 2).

### <Test Example 1-1> Gel forming ability test

With respect to Example 1 prepared in Preparation Example 1-1 and Example 2 prepared in Preparation Example 1-2, the gel forming ability at 37°C was evaluated. After 300 µL of each base liquid was dropped onto a plastic Petri dish (1000-035, manufactured by IWAKI), the dish was covered with a lid, and placed on the water surface of a water bath (TR-2A, manufactured by AS ONE Corporation) set to 37°C. After 10 seconds, 15 seconds, or 20 seconds from the beginning of the placement, each plastic Petri dish was taken out and immediately inverted to visually observe the behavior of the base liquid for 10 seconds from the inversion. Each sample was measured at N=2. The case where "dropping" or "flowing horizontally" was observed in less than 5 seconds was evaluated as "×", and the case where "dropping" and "flowing horizontally" were not observed for at least 5 seconds was evaluated as "∘". The results are shown in Table 1.

**[Table 1]**

| | Base liquid | Gel forming ability (37°C) | | |
|---|---|---|---|---|
| | | Placing time | | |
| | | 10 sec. | 15 sec. | 20 sec. |
| Example 1 | 3.8% MCC-CMCNa | ○ | ○ | ○ |
| Example 2 | 17.0% Plu. -PBS | × | ○ | ○ |

Since 3.8% MCC·CMCNa (Example 1) tended to form a gel faster than 17.0% Plu.-PBS base liquid (Example 2), the retention in the ear can be expected.

### <Preparation Example 1-3> Preparation of 1.0% to 9.1% MCC CMCNa base liquids

By a method similar to that in Preparation Example 1-1 or by a method to dilute the prepared base liquids with an appropriate amount of purified water, 1.0%, 1.5%, 1.7%, 1.9%, 2.4%, 2.6%, 2.9%, 3.8%, 4.8%, 5.7%, 6.5%, 7.4%, 8.3%, and 9.1% MCC·CMCNa base liquids (Example 3 to 16) were prepared. In connection with this, an MCC·CMCNa base liquid having a concentration higher than 9.1% could not be prepared, because MCC·CMCNa could not be properly dispersed in purified water.

### <Test Example 1-2> Gel forming ability test

With respect to Examples 3 to 16 prepared in Preparation Example 1-3, the gel forming ability at 37°C was evaluated. The test conditions were the same as those in Test Example 1-1, except that the placing time was changed to 10 minutes and that the dropping amount was changed to 300, 200 µL, 100 µL, and/or 50 µL. Each sample was measured at N=2. The case where "dropping" or "flowing horizontally" was observed in less than 5 seconds was evaluated as "×", and the case where "dropping" and "flowing horizontally" were not observed for at least 5 seconds was evaluated as "∘". The case where the test was not carried out was represented by "-". The results are shown in Table 2.

**[Table 2]**

| | MCC·CMCNa conc. (%) | Dropping amount | | | |
|---|---|---|---|---|---|
| | | 300 µL | 200 µL | 100 µL | 50 µL |
| Example 3 | 1.0 | × | × | × | × |
| Example 4 | 1.5 | × | × | × | ○ |
| Example 5 | 1.7 | × | × | × | ○ |
| Example 6 | 1.9 | × | × | ○ | ○ |
| Example 7 | 2.4 | - | × | ○ | ○ |
| Example 8 | 2.6 | ○ | ○ | ○ | ○ |
| Example 9 | 2.9 | ○ | ○ | ○ | ○ |
| Example 10 | 3.8 | ○ | ○ | ○ | ○ |
| Example 11 | 4.8 | ○ | - | - | - |
| Example 12 | 5.7 | ○ | - | - | - |
| Example 13 | 6.5 | ○ | - | - | - |
| Example 14 | 7.4 | ○ | - | - | - |
| Example 15 | 8.3 | ○ | - | - | - |
| Example 16 | 9.1 | ○ | - | - | - |

Under the conditions that the dropping amount was 50 µL, MCC·CMCNa base liquids having a concentration of 1.5% or more formed a gel temporarily or continuously. Similarly, under the conditions of a dropping amount of 100 µL, MCC·CMCNa base liquids having a concentration of 1.9% or more formed a gel temporarily or continuously; under the conditions of a dropping amount of 200 µL, MCC·CMCNa base liquids having a concentration of 2.6% or more formed a gel temporarily or continuously; and under the conditions of a dropping amount of 300 µL, MCC·CMCNa base liquids having a concentration of 2.6% or more formed a gel temporarily or continuously.

### Experiment 2: Evaluation of fluidity of base liquids

### <Test Example 2> Fluidity test for base liquids

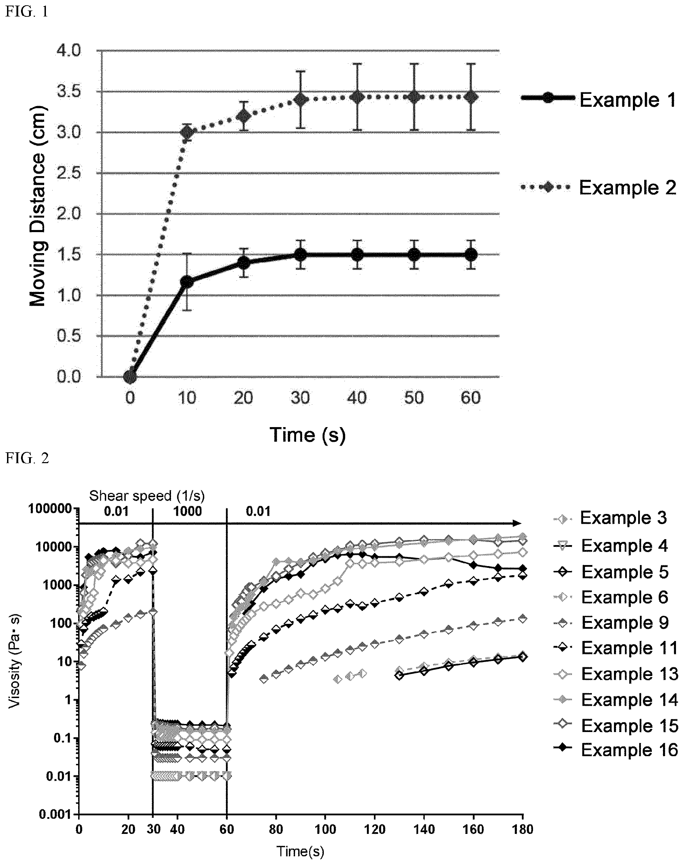

With respect to Example 1 prepared in Preparation Example 1-1 and Example 2 prepared in Preparation Example 1-2, the fluidity was evaluated. In a chromatographic chamber (M-600FN, manufactured by TAITEC) with a set temperature of 37°C, a stainless-steel square vat was placed so that the angle between the bottom surface and the horizontal was 30 degrees, and 0.5 mL of each base liquid was gently dropped onto the bottom surface of the square vat. For 60 seconds after the dropping, the moving distance of the base liquid flowed down the bottom surface of the square vat was measured every 10 seconds (N=3). The results are shown in Figure 1. The error bar in Figure 1 means standard deviation.

Since the 3.8% MCC·CMCNa base liquid (Example 1) had a shorter moving distance than the 17.0% Plu.-PBS base liquid (Example 2), it can be expected that the drug can be more retained for a long time in the ear.

### Experiment 3: Evaluation of dosing pressure

Base liquids having a viscosity equal to or higher than a certain standard were prepared using pharmaceutical additives for imparting viscosity, and the maximum dosing pressure applied at the time of injection administration was measured.

### <Preparation Example 3-1> Preparation of each base liquid

Sodium hyaluronate, sodium alginate, CMC-Na, MC, HEC, HPC, HPMC, polyvinylpyrrolidone, pectin, carbopol, carrageenan, caraya gum, and xanthan gum were used as the pharmaceutical additives for imparting viscosity, and each pharmaceutical additive was added to purified water and dissolved or the like so as to have the base concentrations shown in Table 3 to prepare base liquids of Examples 17 to 29. The concentration of each of the above bases was set so as to have a complex viscosity of 1650 mPa·s or more at the time of temperature rise of 37°C, when a measurement was carried out using a rheometer (all rheometers used in the Examples were MCR302, manufactured by Anton Paar.) equipped with a plate (MEASURING CONE CP25-2, manufactured by Anton Paar), a hood (all hoods used in the Examples were H-PTD200, manufactured by Anton Paar.), and a gap of 0.3 mm, under conditions of a sample volume of 0.3 mL, a shear strain of 5%, an angular frequency of 50 rad/sec., and a normal force of 0 N, and under temperature conditions in which the beginning of measurement was 25°C, and the temperature increased was 1°C per 10 seconds, up to 37°C. Although the complex viscosity of the polyvinylpyrrolidone solution decreases with heating, 70.0% polyvinylpyrrolidone was adopted because the complex viscosity at 25°C exceeded 1650 mPa·s. A 17.0% Plu.-PBS base liquid (Example 30) was prepared by adding the base to 1×PBS so as to have the concentration shown in Table 3 and stirring and dissolving it at approximately 4°C. Purified water (Example 31) was used as a base liquid without a pharmaceutical additive for imparting viscosity.

### <Test Example 3-1> Measurement of maximum dosing pressure applied during injection administration (under 25°C conditions)

With respect to some base liquids (Examples 9 to 11) prepared in Preparative Example 1-3 and each of the base liquid liquids (Examples 17 to 31) prepared in Preparative Example 3-1, the maximum dosing pressure applied during injection administration was measured. The measurement of dosing pressure was carried out by setting a load cell (LC2-3305B-200N, contact area: 25 mm², manufactured by YAMADEN) in RHEONER2 CREEPMETER (RE2-3305C, manufactured by YAMADEN), and controlling the temperature of measurement environment at 25°C using a precision air conditioner (PAU-300S-HC, manufactured by Apiste). One mL of each base liquid of Examples 9 to 11 or 17 to 30 or purified water (Example 31) was filled in a luer lock syringe (SOFT-JECT S5010-LL, manufactured by Henke sass wolf), and a 25G injection needle (25G × 60 mm, manufactured by TOP) was attached. After removing air bubbles, the filling liquid volume was adjusted to 0.5 mL. This syringe was set in a measuring device, and the dosing pressure was measured by pushing it at a speed of 1 mm/sec. in the vertical direction (N=3). The results are shown in Table 3. In connection with this, since the load of the dosing pressure in Example 24 was too large and the injection needle came off during the measurement, the maximum dosing pressure is shown as the lowest value among the maximum dosing pressures of N=3, instead of the average value. The case where the test was not carried out was represented by "-".

**[Table 3]**

| | Type and cone. of base liquid | Complex viscosity (mPa·s) | Maximum dosing pressure (N) * |
|---|---|---|---|
| Example 9 | 2.9% MCC CMCNa | - | 2.3±0.1 |
| Example 10 | 3.8% MCC CMCNa | - | 3.0±0.0 |
| Example 11 | 4.8% MCC·CMCNa | - | 4.1±0.1 |
| Example 17 | 2.5%(w/w) Sodium hyaluronate | 6881.3 | 9.9±0.1 |
| Example 18 | 3.0%(w/w) Sodium alginate | 3258.1 | 13.2±0.1 |
| Example 19 | 4.5%(w/w) CMCNa | 3742.7 | 24.8±0.3 |
| Example 20 | 3.0%(w/w) MC | 2820.2 | 19.6±0.4 |
| Example 21 | 2.5%(w/w) HEC | 3675.6 | 10.9±0.1 |
| Example 22 | 4.5%(w/w) HPC | 5064.8 | 18.4±0.3 |
| Example 23 | 3.0%(w/w) HPMC | 3096.7 | 19.0±0.3 |
| Example 24 | 70.0%(w/w) Polyvinylpyrrolidone | 2275.5 | ≧63.7 |
| Example 25 | 5.0%(w/w) Pectin | 3104.4 | 24.2±0.2 |
| Example 26 | 10.0%(w/w) Carbopol | 5258.2 | 48.2±8.0 |
| Example 27 | 3.0%(w/w) Carrageenan | 4966.8 | 9.1±0.6 |
| Example 28 | 3.0%(w/w) Karaya gum | 5818.8 | 5.6±0.1 |
| Example 29 | 5.0%(w/w) Xanthan gum | 3061.7 | 6.9±0.1 |
| Example 30 | 17.0% Plu.-PBS | - | 12.9±0.3 |
| Example 31 | Purified water | - | 0.6±0.1 |

| | | | |
|---|---|---|---|
| * average ± standard deviation | | | |

### <Test Example 3-2> Measurement of maximum dosing pressure applied during injection administration (under 37°C conditions)

With respect to some of the base liquids prepared in Preparation Example 1-3 (Examples 3, 6, and 9 to 16), the maximum dosing pressure during injection administration was measured. The test conditions were the same as those in Test Example 3-1, except for the temperature conditions. As the temperature conditions, to mimic the temperature change during otic administration, the test was carried out in a state in which the entire injection needle connected to the syringe was immersed in water at 37°C±0.5°C (N=3). The results are shown in Table 4.

**[Table 4]**

| | MCC·CMCNa conc. (%) | Maximum dosing pressure (N) * |
|---|---|---|
| Example 3 | 1.0 | 0.9±0.0 |
| Example 6 | 1.9 | 1.2±0.0 |
| Example 9 | 2.9 | 2.1±0.0 |
| Example 10 | 3.8 | 2.6±0.1 |
| Example 11 | 4.8 | 3.7±0.0 |
| Example 12 | 5.7 | 4.7±0.2 |
| Example 13 | 6.5 | 5.3±0.0 |
| Example 14 | 7.4 | 7.3±0.2 |
| Example 15 | 8.3 | 8.2±0.4 |
| Example 16 | 9.1 | 10.1±0.1 |

| | | |
|---|---|---|
| * average ± standard deviation | | |

The maximum dosing pressure in the MCC·CMCNa base liquids with concentrations of 1.0% to 6.5% was approximately 5 N, and the maximum dosing pressure in the MCC·CMCNa base liquid with the maximum concentration of 9.1% was also approximately 10 N, and therefore, it can be expected that otic administration can be easily carried out.

### Experiment 4: Evaluation of viscosity during injection administration <preparation Example 4-1> Measurement of static viscosity and dynamic viscosity during injection administration according to base liquid concentration

With respect to some base liquids (Examples 3 to 6, 9, 11, and 13 to 16) prepared in Preparation Example 1-3, the viscosity over time was measured under conditions assuming injection administration. A rheometer (MCR302, manufactured by Anton Paar) equipped with a plate (MEASURING CONE CP50-1, manufactured by Anton Paar), a hood (H-PTD200, manufactured by Anton Paar), and a gap of 0.3 mm was used under conditions of a sample volume of 1 mL, a normal force of 0 N, a torque reliability range of 1 µN·m or more, and a measurement temperature of 37°C. The shear rate started at 0.01 (1/sec.) in order to mimic the static state), and was changed to 1000 (1/sec.) 30 seconds after the start of measurement, and was returned to 0.01 (1/sec.) 60 seconds after the start of measurement. When the torque reliability range was not exceeded, it was regarded as less than the Limit of Quantitation (LOQ).

The measurement was carried out at N=2, and the average values were obtained. The results are shown in Table 5 and Figure 2.

**[Table 5]**

| | MCC·CMCNa conc. (%) | Dynamic or static viscosity (Pa·s) | | | | |
|---|---|---|---|---|---|---|
| | | 30 sec. | 60 sec. | 120 sec. | 150 sec. | 180 sec. |
| Example 3 | 1.0 | <LOQ | 0.01 | <LOQ | <LOQ | <LOQ |
| Example 4 | 1.5 | <LOQ | 0.01 | <LOQ | <LOQ | <LOQ |
| Example 5 | 1.7 | <LOQ | 0.01 | <LOQ | 7.59 | 13.09 |
| Example 6 | 1.9 | <LOQ | 0.01 | <LOQ | 9.31 | 14.57 |
| Example 9 | 2.9 | 197.99 | 0.03 | 28.41 | 67.53 | 131.47 |
| Example 11 | 4.8 | 2332.50 | 0.05 | 338.03 | 1076.58 | 1763.85 |
| Example 13 | 6.5 | 4645.80 | 0.09 | 3779.70 | 5286.4 | 7143.80 |
| Example 14 | 7.4 | 9496.25 | 0.15 | 9823.65 | 13969 | 18453.00 |
| Example 15 | 8.3 | 12135.50 | 0.17 | 12074.00 | 15019.5 | 14251.00 |
| Example 16 | 9.1 | 7137.10 | 0.21 | 5272.35 | 5179.6 | 2659.85 |
| Shear rate (l/sec.) | | 0.01 | 1000 | 0.01 | 0.01 | 0.01 |

The static viscosity of the MCC·CMCNa base liquid increased in a concentrationdependent manner, and the higher the concentration, the faster the viscosity recovery. When the concentration of the MCC·CMCNa base liquid was 1.7% or more, the viscosity at 120 seconds after the shear rate was returned to 0.01 (1/sec.) (180 seconds after the start of measurement) exceeded 10 Pa s. When the concentration of the MCC·CMCNa base liquid was 2.9% or more, the viscosity at 60 seconds after the shear rate was returned to 0.01 (1/sec.) (120 seconds after the start of measurement) exceeded 10 Pa s. When the concentration of the MCC·CMCNa base liquid exceeded 7.4%, there was a tendency to converge to the same extent. When the concentration of the MCC·CMCNa base liquid was 9.1% or more, there was a tendency for the viscosity to decrease at approximately 120 seconds after the shear rate was returned to 0.01 (1/sec.). When the concentration of the MCC·CMCNa base liquid was 1.5% or less, the viscosity at 120 seconds after the shear rate was returned to 0.01 (1/sec.) (180 seconds after the start of measurement) was equal to or lower than the limit of quantitation.

### <Preparation Example 4-1>

After 0.4 g of MCC·CMCNa was weighed, it was added to 10 g of pure water, and dispersed to prepare a 3.8% MCC·CMCNa base liquid (Example 32). After 4 g of MCC CMCNa was weighed, it was added to 100 g of pure water, and dispersed, and further, the base liquid was pulverized twice under conditions of 100 MPa using a wet-type super atomizer (Nanovater NVL-ED015-D10L-XT110, manufactured by Yoshida Kikai) to prepare a 3.8% MCC CMCNa base liquid (pulverized) (Example 33).

### <Test Example 4-3> Measurement of particle size of dispersed component in each base liquid

The particle size of each base liquid prepared in Preparation Example 4-1 was measured. After adding approximately 20 µL of each base liquid to a measurement cell filled with purified water and stirring it with a mini stirrer, the particle size of dispersion in each base liquid was measured using a particle size distribution analyzer (LA-960V2, manufactured by HORIBA) by a laser diffraction/scattering measurement method. The results are shown in Table 7.

### <Test Example 4-4> viscosity measurement of each base liquid

The effect of pulverization of MCC·CMCNa on viscosity was investigated. Viscosity change was measured under conditions assuming injection administration of each base liquid prepared in Preparation Example 4-1. The test conditions were the same as those in Test Example 4-2, except for the number of measurements (N=3). The results are shown in Table 6.

**[Table 6]**

| | Base liquid | Particle size (µm) | Dynamic or static viscosity (Pa·s)^{∗} | | | |
|---|---|---|---|---|---|---|
| | | | 30 sec. | 60 sec. | 120 sec. | 180 sec. |
| Example 32 | 3.8% MCC·CMCNa | 64.5 | 246.74 ±157.36 | 0.04 ±0.00 | 137.31 ±39.16 | 216.78 ±98.04 |
| Example 33 | 3.8% MCC CMCNa (pulverized) | 5.8 | 330.19 ±21.74 | 0.03 ±0.00 | 268.72 ±13.15 | 758.64 ±49.28 |
| Shear rate (1/sec.) | | | 0.01 | 1000 | 0.01 | 0.01 |

| | | | | | | |
|---|---|---|---|---|---|---|
| * average ± standard deviation | | | | | | |

### Experiment 5: Effect of DMSO addition on viscosity

Assuming that a drug is poorly water-soluble, the viscosity or the like of base liquids when DMSO as a solubilizer was further added was evaluated.

### <Preparation Example 5-1> Preparation of MCC CMCNa base liquids (with/without DMSO)

After 0.33 g, 0.44 g, and 0.55 g of MCC·CMCNa were weighed, each was added to 10 mL of purified water and stirred to prepare 3.2%, 4.2%, and 5.2% MCC·CMCNa base liquids. Each of these base liquids and purified water were mixed at a ratio of 9:1, or each base liquid, purified water, and DMSO were mixed at a ratio of 9:0.5:0.5 to prepare a 2.9% MCC·CMCNa base liquid (Example 34) and a 5.0%(v/v) DMSO-containing 2.9% MCC·CMCNa base liquid (Example 35), 3.8% MCC CMCNa base liquid (Example 36) and 5.0%(v/v) DMSO-containing 3.8% MCC CMCNa base liquid (Example 37), and 4.7% MCC CMCNa base liquid (Example 38) and 5.0%(v/v) DMSO-containing 4.7% MCC·CMCNa base liquid (Example 39).

### <Preparation Example 5-2> Preparation of Plu. base liquids (with/without DMSO)

After 1.44 g, 1.67 g, and 1.89 g of Plu. were weighed, each was added to 8.56 g, 8.33 g, and 8.11 g of 1×PBS, and stirred and dissolved at approximately 4°C to prepare 14.4%, 16.7%, and 18.9% Plu.-PBS base liquids. Each of these base liquids and PBS were mixed at a ratio of 9:1, or each base liquid, PBS, and DMSO were mixed at a ratio of 9:0.5:0.5 to prepare 13.0% Plu.-PBS base liquid (Example 40) and 5.0%(v/v) DMSO-containing 13.0% Plu.-PBS base liquid (Example 41), 15.0% Plu.-PBS base liquid (Example 42) and 5.0%(v/v) DMSO-containing 15.0% Plu.-PBS base liquid (Example 43), and 17.0% Plu.-PBS base liquid (Example 44) and 5.0%(v/v) DMSO-containing 17.0% Plu.-PBS base liquid (Example 45).

### <Test Example 5-1> Viscosity measurement test of MCC CMCNa base liquids (with/without DMSO)

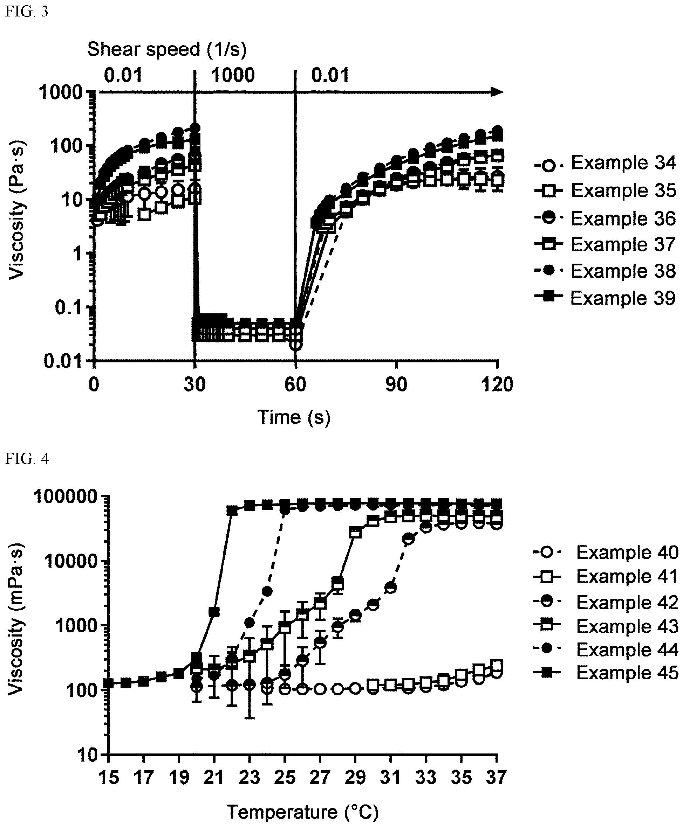

With respect to each of the MCC CMCNa base liquids (Examples 34 to 39) prepared in Preparation Example 5-1, the change in viscosity due to the shear rate was measured. The test conditions were the same as those in Test Example 4-2, except for the number of measurements (N=3) and the measurement time (measurement for 60 seconds after the shear rate was returned to 0.01 (1/sec.)). The results are shown in Table 7 and Figure 3.

**[Table 7]**

| | MCC·CMC Na Conc. (%) | DMSO Conc. (%) | Dynamic or static viscosity (Pa·s)^{∗} | | |
|---|---|---|---|---|---|
| | | | 30 sec. | 60 sec. | 120 sec. |
| Example 34 | 2.9 | - | 15.69±7.34 | 0.02±0.00 | 26.75±12.22 |
| Example 35 | | 5 | 10.50±2.11 | 0.03±0.00 | 22.63±0.59 |
| Example 36 | 3.8 | - | 65.17±17.88 | 0.04±0.00 | 65.47±9.44 |
| Example 37 | | 5 | 42.99±5.01 | 0.04±0.00 | 65.91±4.95 |
| Example 38 | 4.7 | - | 212.26±23.64 | 0.05±0.00 | 189.46±10.73 |
| Example 39 | | 5 | 128.52±34.95 | 0.05±0.00 | 149.26±3.69 |
| Shear rate (1/sec.) | | | 0.01 | 1000 | 0.01 |

| | | | | | |
|---|---|---|---|---|---|
| ^{∗} average ± standard deviation | | | | | |

### <Test Example 5-2> Complex viscosity measurement test of Plu. base liquids (with/without DMSO)

With respect to each of the Plu. base liquids (Examples 40 to 45) prepared in Preparation Example 5-2, the complex viscosity over time due to temperature change, and the sol-gel transition point were measured (N=3). The term "complex viscosity" as used herein means a viscosity obtained by dynamic measurement using a rheometer. The term "sol-gel transition point" means the temperature at which a pharmaceutical composition changes from a sol state to a gel state. The change in complex viscosity and the sol-gel transition point were measured using a rheometer equipped with a plate (MEASURING CONE CP25-2, manufactured by Anton Paar), a hood, and a gap of 0.3 mm, under conditions of a sample volume of 0.3 mL, a shear strain of 5%, an angular frequency of 50 rad/sec., a normal force of 0 N, and a torque reliability range of 1 µN·m or more, and under temperature conditions in which the beginning of measurement was set to 15°C or 20°C, and the temperature increased was 1°C per 10 seconds, up to 37°C. The results are shown in Table 8 and Figure 4.

**[Table 8]**

| | Plu. conc. (%) | DMSO conc. (%) | Complex viscosity (mPa·s)^{∗} | |
|---|---|---|---|---|
| | | | Temperature at which complex viscosity reached 10000 mPa·s (°C) | Sol-gel transition point (°C) |
| Example 40 | 13.0 | - | >37 | >37 |
| Example 41 | | 5 | >37 | >37 |
| Example 42 | 15.0 | - | 31-32 | 27.3±1.0 |
| Example 43 | | 5 | 28-29 | 24.4±1.1 |
| Example 44 | 17.0 | - | 24-25 | 22.8±0.1 |
| Example 45 | | 5 | 21-22 | 20.7±0.1 |

| | | | | |
|---|---|---|---|---|
| ^{∗} average ± standard deviation | | | | |

In the MCC·CMCNa base liquids, no significant change in viscosity was observed with or without DMSO at any concentration. Furthermore, there was no difference in the viscosity change rate with respect to the shear rate fluctuation. On the other hand, when 15.0% Plu.-PBS base liquid was compared with 5.0%(v/v) DMSO-containing 15.0% Plu.-PBS base liquid, the temperature at which the complex viscosity reached 10000 mPa·s and the sol-gel transition point were shifted to the low temperature side by 2°C to 3°C by containing DMSO, and similar results were observed with the 17.0% Plu.-PBS base liquids. With respect to the 13.0% Plu.-PBS base liquids, an increase in complex viscosity and the sol-gel transition, due to an increase in temperature, were not observed, regardless of the presence or absence of DMSO. It was clarified from these results that the viscosity of the MCC·CMCNa base liquid was not affected by DMSO.

### Experiment 6: Evaluation of sustained drug release

With respect to drug-containing liquid preparations containing MCC·CMCNa as the base liquid, the sustained drug release was evaluated using a plurality of model drugs. As the model drugs, FITC dextran (10 kDa, 70 kDa, and 150 kDa) (hereinafter also referred to as FITC-DEX) as a drug model of proteins, acetaminophen and diclofenac sodium as drug models of acidic small molecules with different partition coefficients, and nicardipine hydrochloride as a drug model of basic small molecules were used.

### <Preparation Example 6-1> Preparation of FITC-DEX (10 kDa)-containing liquid preparations

A 1.0%(w/v) FITC-DEX was prepared by dissolving 50 mg of FITC-DEX (10 kDa) in a D-PBS(-) solution and adjusting the volume to 5 mL. A 0.1% FITC-DEX-containing liquid preparation (Example 46) was prepared by mixing 200 µL of 1.0%(w/v) FITC-DEX in 1800 µL of D-PBS(-).

After 945 mg of Plu. was weighed, it was added to 4.055 g of 1×PBS, and dissolved at approximately 4°C to prepare a 18.9% Plu.-PBS base liquid. A 0.1% FITC-DEX-containing 17.0% Plu.-PBS liquid preparation (Example 47) was prepared by mixing 200 µL of 1.0%(w/v) FITC-DEX in 1800 µL of the base liquid.

After 0.11 g of MCC·CMCNa was weighed, it was added to 10 g of purified water, and dispersed to prepare a 1.1% MCC·CMCNa base liquid. A 0.1% FITC-DEX-containing 1.0% MCC·CMCNa liquid preparation (Example 48) was prepared by mixing 200 µL of 1.0%(w/v) FITC-DEX in 1800 µL of the base liquid. Similarly, 0.1% FITC-DEX-containing 1.5% MCC• CMCNa liquid preparation (Example 49), 0.1% FITC-DEX-containing 1.7% MCC• CMCNa liquid preparation (Example 50), 0.1% FITC-DEX-containing 1.9% MCC•CMCNa liquid preparation (Example 51), 0.1% FITC-DEX-containing 2.9% MCC•CMCNa liquid preparation (Example 52), 0.1% FITC-DEX-containing 3.8% MCC·CMCNa liquid preparation (Example 53), and 0.1% FITC-DEX-containing 4.7% MCC•CMCNa liquid preparation (Example 54) were prepared by mixing 200 µL of 1.0%(w/v) FITC-DEX in 1800 µL of each of 1.6% MCC·CMCNa base liquid, 1.9% MCC•CMCNa base liquid, 2.2% MCC•CMCNa base liquid, 3.2% MCC•CMCNa base liquid, 4.2% MCC•CMCNa base liquid, and 5.2% MCC•CMCNa base liquid.

### <Test Example 6-1> Dissolution test of FITC-DEX (10 kDa)-containing liquid preparations

With respect to each of the liquid preparations (Examples 46 to 54) prepared in Preparation Example 6-1, a dissolution test was carried out (N=2). The dissolution test was carried out by putting 2 mL of D-PBS into the lower side of each well of Trans well (6 well, 12 mm diameter inserts, 0.4 µm pore size, manufacturing by Corning), heating the Trans well in a chromatographic chamber (M-600FN, manufactured by TAITEC) set at 37°C, adding 0.5 mL of each liquid preparation to the upper side of each well, and shaking it at 37°C ± 2°C and 100 rpm. At 1 hour, 2 hours, 4 hours, 6 hours, and 24 hours after the beginning of the test, 0.5 mL of aliquot was sampled from the lower side of each well, and 0.5 mL of D-PBS was added to the lower side of each well. The FITC concentration in each sample liquid was measured using a fluorescent plate reader (SpectraMax i3x, manufactured by MOLECLTLAR DEVICES) at an excitation wavelength of 494 nm and a measurement wavelength of 522 nm to calculate the dissolution rate. The results are shown in Table 9.

**[Table 9]**

| | FITC-DEX (10kDa)-containing liquid preparation | Dissolution rate (%) | | | | |
|---|---|---|---|---|---|---|
| | | 1 hr. | 2 hr. | 4 hr. | 6 hr. | 24 hr. |
| Example 46 | PBS | 53.9 | 66.9 | 73.5 | 76.7 | 79.5 |
| Example 47 | 17.0%Plu.-PBS | 9.3 | 15.6 | 28.1 | 37.5 | 43.4 |
| Example 48 | 1.0% MCC·CMCNa | 18.0 | 25.1 | 34.0 | 43.8 | 74.0 |
| Example 49 | 1.5% MCC·CMCNa | 15.5 | 22.4 | 32.1 | 38.5 | 72.1 |
| Example 50 | 1.7% MCC·CMCNa | 14.0 | 23.1 | 31.6 | 34.3 | 69.7 |
| Example 51 | 1.9% MCC·CMCNa | 14.7 | 19.3 | 29.8 | 38.0 | 69.9 |
| Example 52 | 2.9% MCC·CMCNa | 11.9 | 17.4 | 26.4 | 30.2 | 60.1 |
| Example 53 | 3.8% MCC·CMCNa | 10.4 | 15.3 | 22.4 | 28.4 | 61.3 |
| Example 54 | 4.7% MCC·CMCNa | 10.4 | 15.7 | 21.1 | 27.7 | 63.4 |

### <Preparation Example 6-2> Preparation of FITC-DEX (70 kDa)-containing liquid preparations

A 1.0%(w/v) FITC-DEX was prepared by dissolving 50 mg of FITC-DEX (70 kDa) in a D-PBS(-) solution and adjusting the volume to 5 mL. The liquid preparations (Examples 55 and 56) shown in Table 10 were prepared by a similar method to that in Preparation Example 6-1.

After 9.9 g of MCC·CMCNa was weighed, it was added to 290.1 g of purified water, and dispersed to prepare a 3.3% MCC·CMCNa base liquid. A 0.1% FITC-DEX-containing 3.0% MCC·CMCNa liquid preparation (Example 57) was prepared by mixing 200 µL of 1.0%(w/v) FITC-DEX in 1800 µL of the base liquid. Similarly, 0.1% FITC-DEX-containing 4.0% MCC• CMCNa liquid preparation (Example 58) and 0.1% FITC-DEX-containing 5.0% MCC. CMCNa liquid preparation (Example 59) were prepared by mixing 200 µL of 1.0%(w/v) FITC-DEX in 1800 µL of each of 4.4% MCC•CMCNa base liquid and 5.5% MCC•CMCNa base liquid.

### <Test Example 6-2> Dissolution test of FITC-DEX (70 kDa)-containing liquid preparations

With respect to Examples 55 to 59 prepared in Preparation Example 6-2, a dissolution test of FITC-DEX (70 kDa) was carried out under the same test conditions and method as those in Test Example 6-1 except that using another fluorescent plate reader (Infinite M1000PROSpectraMax i3x, manufactured by MOLECULAR DEVICES) (N=2). The results are shown in Table 10.

**[Table 10]**

| | FITC-DEX (70kDa)-containing liquid preparation | Dissolution rate (%) | | | | |
|---|---|---|---|---|---|---|
| | | 1 hr. | 2 hr. | 4 hr. | 6 hr. | 24 hr. |
| Example 55 | PBS | 42.6 | 58.7 | 72.0 | 77.9 | 83.8 |
| Example 56 | 17.0%Plu.-PBS | 15.2 | 26.3 | 45.7 | 58.3 | 69.6 |
| Example 57 | 3.0% MCC·CMCNa | 9.2 | 14.3 | 22.5 | 29.0 | 73.7 |
| Example 58 | 4.0% MCC·CMCNa | 10.9 | 17.6 | 26.1 | 32.1 | 65.1 |
| Example 59 | 5.0% MCC·CMCNa | 9.2 | 15.3 | 25.5 | 31.2 | 73.1 |

### <Preparation Example 6-3> Preparation of FITC-DEX (150 kDa)-containing liquid preparations

A 1.0%(w/v) FITC-DEX was prepared by dissolving 50 mg of FITC-DEX (150 kDa) in a D-PBS(-) solution and adjusting the volume to 5 mL. The liquid preparations (Examples 60 to 64) shown in Table 11 were prepared by a similar method to that in Preparation Example 6-2.

### <Test Example 6-3> Dissolution test of FITC-DEX (150 kDa)-containing liquid preparations

With respect to Examples 60 to 64 prepared in Preparation Example 6-3, a dissolution test of FITC-DEX (150 kDa) was carried out under the same test conditions and method as those in Test Example 6-1 (N=2). The results are shown in Table 11.

**[Table 11]**

| | FITC-DEX (150kDa)-containing liquid preparation | Dissolution rate (%) | | | | |
|---|---|---|---|---|---|---|
| | | 1 hr. | 2 hr. | 4 hr. | 6 hr. | 24 hr. |
| Example 60 | PBS | 26.7 | 47.3 | 71.6 | 74.6 | 88.5 |
| Example 61 | 17.0% Plu.-PBS | 15.9 | 30.1 | 52.6 | 63.0 | 79.0 |
| Example 62 | 3.0% MCC·CMCNa | 12.7 | 19.2 | 29.9 | 39.0 | 79.2 |
| Example 63 | 4.0% MCC CMCNa | 10.8 | 17.7 | 26.7 | 31.9 | 72.09 |
| Example 64 | 5.0% MCC·CMCNa | 9.0 | 14.7 | 22.6 | 29.5 | 64.2 |

### <Preparation Example 6-4> Preparation of acetaminophen-containing liquid preparations

After 100 mg of acetaminophen was weighed, it was dissolved in purified water and adjusted to 10 mL to prepare a 1.0%(w/v) acetaminophen. A 0.1%(w/v) acetaminophen-containing PBS liquid preparation (Example 65) was prepared by mixing 120 µL of 1.0%(w/v) acetaminophen in 1080 µL of 1×PBS.

After 100 mg of acetaminophen was weighed, it was dissolved in 0.05% polysorbate 20/PBS solution and adjusted to 10 mL to prepare a 1.0%(w/v) acetaminophen/PBS solution. After 18.9 g of Plu. was weighed, it was added to 81.1 g of 1 ×PBS, and dissolved at approximately 4°C to prepare a 18.9% Plu.-PBS base liquid. A 0.1% acetaminophen-containing 17.0% Plu.-PBS liquid preparation (Example 66) was prepared by mixing 300 µL of 1.0%(w/v) acetaminophen/PBS solution in 2700 µL of the base liquid.

After 2.2 g of MCC·CMCNa was weighed, it was dispersed in 50 mL of purified water to prepare a 4.2% MCC·CMCNa base liquid. A 0.1% acetaminophen-containing 3.8% MCC·CMCNa liquid preparation (Example 67) was prepared by mixing 150 µL of 1.0%(w/v) acetaminophen in 1350 µL of the base liquid.

### <Test Example 6-4> Dissolution test of acetaminophen-containing liquid preparations

With respect to Examples 65 to 67 prepared in Preparation Example 6-4, a dissolution test was carried out under the same test conditions as those in Test Example 6-1, except that the dissolution test liquid was changed to 1×PBS (N=2). The acetaminophen concentration in each sample liquid was measured using HPLC (ESPRIMO D583/N, manufactured by Waters) at a measurement wavelength of 254 nm. The results are shown in Table 12.

**[Table 12]**

| | Acetaminophen-containing liquid preparation | Dissolution rate (%) | | |
|---|---|---|---|---|
| | | 1 hr. | 6 hr. | 24 hr. |
| Example 65 | PBS | 77.0 | 91.6 | 95.9 |
| Example 66 | 17.0%Plu.-PBS | 28.1 | 82.1 | 100.0 |
| Example 67 | 3.8% MCC-CMCNa | 31.3 | 75.2 | 89.8 |

### <Preparation Example 6-5> Preparation of diclofenac sodium-containing liquid preparations

A 1.0%(w/v) diclofenac sodium was prepared by dissolving 50 mg of diclofenac sodium in D-PBS(-) and adjusting the volume to 5 mL. The liquid preparations (Examples 68 to 70) shown in Table 13 were prepared by a similar method to that in Preparation Example 6-1.

### <Test Example 6-5> Dissolution test of diclofenac sodium-containing solution

With respect to Examples 68 to 70 prepared in Preparation Example 6-5, the dissolution test was carried out under the same test conditions as those in Test Example 6-1. The diclofenac sodium concentration in each sample liquid was measured using an HPLC (Waters Alliance e2695, manufactured by Waters) at a measurement wavelength of 240 nm. The results are shown in Table 13.

**[Table 13]**

| | Diclofenac sodium-containing liquid preparation | Dissolution rate (%) | | | | |
|---|---|---|---|---|---|---|
| | | 1 hr. | 2 hr. | 4 hr. | 6 hr. | 24 hr. |
| Example 68 | PBS | 76.9 | 83.8 | 86.5 | 89.2 | 93.2 |
| Example 69 | 17.0%Plu.-PBS | 15.0 | 27.9 | 50.4 | 66.9 | 92.0 |
| Example 70 | 4.0% MCC CMCNa | 35.2 | 49.9 | 66.4 | 76.3 | 88.9 |

### <Preparation Example 6-6> Preparation of nicardipine hydrochloride-containing liquid preparations

A 0.5%(w/v) nicardipine hydrochloride was prepared by dissolving 25 mg of nicardipine hydrochloride in purified water, and adjusting the volume to 5 mL. A 0.05% nicardipine hydrochloride-containing physiological saline (Example 71) was prepared by mixing 200 µL of 0.5%(w/v) nicardipine hydrochloride into 1800 µL of physiological saline.

A 0.05% nicardipine hydrochloride-containing 17% Plu.-PBS liquid preparation (Example 72) was prepared by mixing 200 µL of 0.5%(w/v) nicardipine hydrochloride into 1800 µL of 18.9% Plu.-PBS base liquid prepared by a method similar to that in Preparation Example 6-1.

A 0.05% nicardipine hydrochloride-containing 4% MCC·CMCNa liquid preparation (Example 73) was prepared by mixing 200 µL of 0.5%(w/v) nicardipine hydrochloride into 1800 µL of 4% MCC·CMCNa base liquid prepared by a method similar to that in Preparation Example 6-1.

### <Test Example 6-6> Dissolution test of nicardipine hydrochloride-containing liquid preparations

With respect to Examples 71 to 73, the dissolution test was carried out under the same test conditions as those in Test Example 6-1, except that the dissolution test solution was physiological saline instead of D-PBS(-). The nicardipine hydrochloride concentration in each sample liquid was measured using an HPLC (Waters Alliance e2695, manufactured by Waters) at a measurement wavelength of 254 nm. The results are shown in Table 14.

**[Table 14]**

| | Nicardipine hydrochloride-containing liquid preparation | Dissolution rate (%) | | | | |
|---|---|---|---|---|---|---|
| | | 1 hr. | 2 hr. | 4 hr. | 6 hr. | 24 hr. |
| Example 71 | Physiological saline | 77.3 | 79.8 | 83.9 | 84.9 | 86.4 |
| Example 72 | 17.0%Plu.-PBS | 15.9 | 28.5 | 59.1 | 77.8 | 94.5 |
| Example 73 | 4.0% MCC-CMCNa | 21.7 | 30.6 | 48.2 | 57.6 | 75.3 |

### Experiment 7: Evaluation of retention properties in ear using fluorescent dye

With respect to the pharmaceutical compositions containing MCC·CMCNa, retention properties in the ear were evaluated using FITC-DEX (10 kDa) as the model drug.

### <Preparation Example 7-1> Preparation of FITC-DEX (10 kDa)-containing liquid preparations

A 1.4%(w/v) FITC-DEX solution was prepared by dissolving 70 mg of FITC-DEX (10 kDa) in 1×PBS and adjusting the volume to 5 mL. A 0.14%(w/v) FITC-DEX-containing PBS liquid preparation (Example 74) was prepared by mixing 400 µL of this solution and 3600 µL of PBS.

After 1.89 g of Plu. was weighed, it was added to 8.11 g of 1×PBS, and dissolved at approximately 4°C to prepare a 18.9% Plu.-PBS base liquid. A 0.14%(w/v) FITC-DEX-containing 17.0%(w/v) Plu. liquid preparation (Example 75).

After 0.11 g of MCC·CMCNa was weighed, it was dispersed in 10 g of purified water to prepare 1.1% MCC CMCNa base liquid. A 0.14%(w/v) FITC-DEX-containing 1.0% MCC CMCNa liquid preparation (Example 76) was prepared by mixing 3600 µL of this base liquid and 400 µL of 1.4%(w/v) FITC-DEX solution. Similarly, a 0.14%(w/v) FITC-DEX-containing 1.9% MCC CMCNa liquid preparation (Example 77), 0.14%(w/v) FITC-DEX-containing 2.9% MCC CMCNa liquid preparation (Example 78), and 0.14%(w/v) FITC-DEX-containing 3.8% MCC·CMCNa liquid preparation (Example 79) by mixing 400 µL of 1.4%(w/v) FITC-DEX into 3600 µL of each of 2.2% MCC CMCNa base liquid, 3.2% MCC·CMCNa base liquid, and 4.2% MCC CMCNa base liquid.

### <Test Example 7-2> Retention test of FITC-DEX (10 kDa)-containing liquid preparations

With respect to each of the liquid preparations (Examples 74 to 79) prepared in Preparation Example 7-1, a retention test in the rat ear was carried out (N=3-5). Under isoflurane (manufactured by Pfizer) inhalation anesthesia, the tympanic membrane of the right ear of each 6-week-old rat (male Slc: SD, Japan SLC) was perforated using a 25G injection needle (NN-2525R, manufactured by Terumo). Through this perforation, 25 µL or 50 µL of each liquid preparation was administered using a gastight syringe (1710LT, manufactured by Hamilton) with a 25G injection needle with an obtuse-angled tip. After administration, the rats were left in the lateral decubitus position for about 10 minutes, the rats were awakened. After 1 or 3 days, the rats were subjected to exsanguination treatment and decapitation under anesthesia, and the middle ear cavity was extracted. FITC-DEX in the extracted middle ear cavity was collected, and the residual amount of FITC-DEX in the ear was measured using a fluorescent plate reader (Infinite M1000PRO, manufactured by TECAN). The results are shown in Table 15.

**[Table 15]**

| | FITC-DEX (10kDa) -containing liquid preparation | Residual rate (%)* | | |
|---|---|---|---|---|
| | | After 1 day | | After 3 days |
| | | 25µL | 50µL | 50µL |
| Example 74 | PBS | 7.0±2.2 | 4.1±0.9 | 2.5±0.7 |
| Example 75 | 17.0% Plu.-PBS | 27.4±7.1 | 34.6±19.6 | 8.8±2.4 |
| Example 76 | 1.0% MCC·CMCNa | 30.1±2.0 | 49.2±5.4 | 25.4±3.5 |
| Example 77 | 1.9% MCC·CMCNa | 42.2±7.7 | 53.5±4.7 | 30.0±5.1 |
| Example 78 | 2.9% MCC·CMCNa | 51.8±3.5 | 67.3±0.9 | 42.6±20.9 |
| Example 79 | 3.8% MCC·CMCNa | 81.4±3.5 | 79.7±0.7 | 56.6±11.4 |

| | | | | |
|---|---|---|---|---|
| * average ± standard deviation | | | | |

### Experiment 8: Evaluation of efficacy ofHB-EGF-containing liquid preparations using chronic tympanic membrane perforation model

A mouse model of chronic tympanic membrane perforation was prepared, and the efficacy for chronic tympanic membrane perforation was evaluated when each mouse HB-EGF-containing liquid preparation was administered.

### <Test Example 8-1> Preparation of mouse model of chronic tympanic membrane perforation

Under isoflurane (manufactured by Pfizer) anesthesia, the right and left tympanic membranes of mouse (male CBA/CaJ species, 6 weeks old) were subjected to total perforation using a Rosen probe (52-147-50, manufactured by DAIICHI MEDICAL). GELFOAM (registered trademark) (manufactured by Pfizer) soaked with a 10 mmol/L KB-R7785 solution (in-house synthesized) (Hirayama et al., Bioorg Med Chem, 1997, Apr; 5(4) 765-778) as a perforating agent was placed through and onto the created perforation. In connection with this, the GELFOAM (registered trademark) was used after being cut into a size that could go into the ear of the mouse. The inside of the ear was observed once a day, and in a case where the GELFOAM (registered trademark) had disappeared, fresh GELFOAM (registered trademark) soaked with the perforating agent was newly placed. In a case where the GELFOAM was retained without disappearing, the 10 mmol/L KB-R7785 solution was additionally administered. The treatment with the perforating agent was carried out for 7 days, subsequently the perforation was left alone for three months, and the perforation was checked with a microscope. Animals that spontaneously healed were omitted from this study.

### <Preparation Example 8-1> Preparation of mouse HB-EGF solution

Mouse HB-EGF (cyt-068, manufactured by Prospec) was used as HB-EGF, and was dissolved in purified water to prepare a mouse HB-EGF solution. To 400 µL of 58.824 µg/mL mouse HB-EGF solution, 70 µL of distilled water was added to adjust the mouse HB-EGF solution to 50 µg/mL.

### <Preparation Example 8-2> Preparation of physiological saline liquid preparations (with/without mouse HB-EGF)

A 5 µg/mL mouse HB-EGF solution (with mouse HB-EGF)(Example 80) or a stilled waterphysiological saline liquid preparation (without mouse HB-EGF)(Example 81) were prepared by diluting the 50 µg/mL mouse HB-EGF solution prepared in Preparation Example 8-1 or stilled water 10-fold with physiological saline. These solutions were stored at 4°C.

### <Preparation Example 8-3> Preparation of 3.8% MCC·CMCNa liquid preparations (with/without mouse HB-EGF)

After 2.2 g of MCC·CMCNa was weighed, it was added to 50 g of purified water, and dispersed to prepare 4.2% MCC·CMCNa base liquid. A 3.8% MCC·CMCNa liquid preparation (with mouse HB-EGF)(Example 82) or a 3.8% MCC·CMCNa liquid preparation (without mouse HB-EGF)(Example 83) were prepared by mixing 50 µg/mL mouse HB-EGF solution prepared in Preparation Example 8-1 or stilled water and 4.2% MCC·CMCNa base liquid at a ratio of 1:9.

### <Test Example 8-2> Treatment of chronic tympanic membrane perforation

To the mouse model of chronic tympanic membrane perforation prepared in Test Example 8-1, 5 µl of each of the liquid preparations prepared in Preparation Examples 8-2 and 8-3 (Examples 80 to 83) was administered onto the tympanic membrane using a Microman E (M10E, manufactured by Gilson) (N=10-12). After one month from the administration, the surrounding tissue including the tympanic membrane was sampled, residue was removed, and then the presence or absence of the perforation was checked. Then, the tympanic membrane perforation healing rate was calculated from the number of perforated tympanic membranes and the number of completely healed tympanic membranes. The results are show in Table 16.

**[Table 16]**

| | Example 80 | Example 81 | Example 82 | Example 83 |
|---|---|---|---|---|
| | Physiological saline liquid preparation | | 3.8% MCC CMCNa liquid preparation | |
| Mouse HB-EGF (5 µg/mL) | Added | Not added | Added | Not added |
| Number of perforated tympanic membranes | 7 | 7 | 4 | 9 |
| Number of completely healed tympanic membranes | 3 | 3 | 7 | 3 |
| Healing rate (%) | 30 | 30 | 64 | 25 |

The healing rate of tympanic membrane perforation by the physiological saline liquid preparation (with mouse HB-EGF)(Example 80) was 30%, whereas the 3.8% MCC CMCNa liquid preparation (with mouse HB-EGF)(Example 82) was 64%.

### INDUSTRIAL APPLICABILITY

According to the present invention, there can be provided a pharmaceutical composition for otic administration that can be easily administered into the ear and has a function of allowing a drug to be retained and sustain released in the ear.

Although the present invention has been described with reference to specific embodiments, various changes and modifications obvious to those skilled in the art are possible without departing from the scope of the appended claims.

## Claims

1. A pharmaceutical composition for otic administration, comprising one, or two or more drugs and a water-dispersible fine cellulose composition.

2. The pharmaceutical composition according to claim 1, wherein the water-dispersible fine cellulose composition contains microcrystalline cellulose and a water-soluble polymer.

3. The pharmaceutical composition according to claim 2, wherein the water-soluble polymer is carmellose sodium.

4. The pharmaceutical composition according to claim 1, wherein the water-dispersible fine cellulose composition is microcrystalline cellulose-carmellose sodium.

5. The pharmaceutical composition according to any one of claims 1 to 4, further comprising a solvent.

6. The pharmaceutical composition according to claim 5, wherein the solvent is water.

7. The pharmaceutical composition according to claim 5 or 6, wherein a ratio of total weight of the water-dispersible fine cellulose composition to the pharmaceutical composition is 1.7% (w/w) to 9.1% (w/w).

8. The pharmaceutical composition according to claim 5 or 6, wherein a ratio of total weight of the water-dispersible fine cellulose composition to the pharmaceutical composition is 2.9% (w/w) to 9.1% (w/w).

9. The pharmaceutical composition according to any one of claims 5 to 8, wherein the drug is slowly released, when an insert equipped with a permeable membrane is inserted into a plate well containing 2 mL of phosphate buffered saline at 37°C ± 2°C so that the phosphate buffered saline is divided into an upper layer and a lower layer, 0.5 mL of the pharmaceutical composition according to any one of claims 1 to 8 is added onto the insert, and the drug is dissolved by shaking the plate well at a rate at which a water surface of the phosphate buffered saline shakes.

10. The pharmaceutical composition according to any one of claims 1 to 9, further comprising a solubilizer.

11. The pharmaceutical composition according to any one of claims 1 to 10, wherein the drug is one, or two or more compounds selected from the group consisting of a small molecule compound, a nucleic acid, and a protein.

12. The pharmaceutical composition according to any one of claims 1 to 11, wherein the drug is a drug that provides a biological activity of a heparin-binding epidermal growth factor.

13. The pharmaceutical composition according to claim 12, wherein the drug that provides a biological activity of a heparin-binding epidermal growth factor comprises a protein consisting of the amino acid sequence of SEQ ID NO: 1.

14. A method of retaining a pharmaceutical composition for otic administration containing one, or two or more drugs into the ear by a water-dispersible fine cellulose composition.

15. The method according to claim 14, wherein the water-dispersible fine cellulose composition is microcrystalline cellulose-carmellose sodium.
